# EUROPEAN PATENT SPECIFICATION

(11) **EP 1 294 902 B1**
(45) Date of publication and mention of the grant of the patent: **13.08.2008**
(21) Application number: 01935595.7
(22) Date of filing: 16.05.2001
(51) Int. Cl.: C12N 15/57, C12N 15/11, C12N 9/64, C12N 9/00, C07K 16/40, C12Q 1/68, G01N 33/53, G01N 33/68

(54) **TREATMENT METHODS USING 17906 AND USES THEREFORE**
BEHANDLUNGSVERFAHREN UNTER VERWENDUNG VON 17906 UND DESSEN ANWENDUNGEN
METHODES DE TRAITEMENT AVEC 17906 ET UTILISATIONS DE CELLES-CI

(30) Priority: 16.05.2000 US 571689
(43) Date of publication of application: 26.03.2003
(73) Proprietor: MILLENNIUM PHARMACEUTICALS, INC., Cambridge, MA 02139 (US)
(72) Inventor: WHITE, David, Braintree, MA 02148 (US); MACBETH, Kyle, Boston, MA 02118 (US)
(74) Representative: Taylor, Kate Laura
(86) International application number: PCT/US2001/015835
(87) International publication number: WO 2001/088157

(56) References cited:
- EP-A- 0 588 118
- EP-A- 0 931 832
- WO-A-00/09709
- WO-A-00/78961
- WO-A-98/45471
- DATABASE EM_HUM [Online] EMBL; ID AF095719, AC AF095719, 6 January 2000 (2000-01-06) HUANG H ET AL.: "Homo sapiens carboxypeptidase A3 mRNA, complete cds" XP002190954 cited in the application -& HUANG H ET AL.: "Carboxypeptidase A3 (CPA3): A novel gene highly induced by histone deacetylase inhibitors during differentiation of prostate epithelial cancer cells" CANCER RESEARCH, vol. 59, no. 12, 15 June 1999 (1999-06-15), pages 2981-2988, XP002190955 cited in the application
- VENDRELL J ET AL.: "Metallocarboxypeptidases and their protein inhibitors - Structure, function and biomedical properties" BIOCHIMICA ET BIOPHYSICA ACTA. PROTEIN STRUCTURE AND MOLECULAR ENZYMOLOGY, vol. 1477, no. 1-2, 7 March 2000 (2000-03-07), pages 284-298, XP004278900 ISSN: 0167-4838
- SUH J ET AL.: "Zn(II)-Chelating Inhibitors of Carboxypeptidase A" BIOORGANIC & MEDICINAL CHEMISTRY LETTERS, vol. 5, no. 6, 16 March 1995 (1995-03-16), pages 585-588, XP004135705 ISSN: 0960-894X

### SEQUENCE LISTING

<110> Millennium Pharmaceuticals, Inc.
<120> TREATMENT METHODS USING 17906 AND USES THEREFOR
<130> 38155-20012.40
<140> PCT/US 01/15835
   <141> 2001-05-16
<150> US 09/571,689
   <151> 2000-05-16
<160> 3
<170> FastSEQ for Windows Version 4.0
<210> 1
   <211> 2795
   <212> DNA
   <213> Homo sapiens
<220>
   <221> CDS
   <222> (8)...(1273)
<400> 1
   cggggac atg agg tgg ata ctg ttc att ggg gcc ctt att ggg tcc agc        49
           Met Arg Trp Ile Leu Phe Ile Gly Ala Leu Ile Gly Ser Ser
            1               5                  10
   atc tgt ggc cga gaa aaa ttt ttt ggg gac caa gtt ttg agg att aat        97
   Ile Cys Gly Arg Glu Lys Phe Phe Gly Asp Gln Val Leu Arg Ile Asn
    15                  20                  25                  30
   gtc aga aat gga gac gag atc agc aaa ttg agt caa cta gtg aat tca       145
   Val Arg Asn Gly Asp Glu Ile Ser Lys Leu Ser Gln Leu Val Asn Ser
                    35                  40                  45
   aac aac ttg aag ctc aat ttc tgg aaa tct ccc tcc tcc ttc aat cgg       193
   Asn Asn Leu Lys Leu Asn Phe Trp Lys Ser Pro Ser Ser Phe Asn Arg
                50                  55                  60
   cct gtg gat gtc ctg gtc cca tct gtc agt ctg cag gca ttt aaa tcc       241
   Pro Val Asp Val Leu Val Pro Ser Val Ser Leu Gln Ala Phe Lys Ser
            65                  70                  75
   ttc ctg aga tcc cag ggc tta gag tac gca gtg aca att gag gac ctg       289
   Phe Leu Arg Ser Gln Gly Leu Glu Tyr Ala Val Thr Ile Glu Asp Leu
        80                  85                  90
   cag gcc ctt tta gac aat gaa gat gat gaa atg caa cac aat gaa ggg       337
   Gln Ala Leu Leu Asp Asn Glu Asp Asp Glu Met Gln His Asn Glu Gly
    95                 100                 105                 110
   caa gaa cgg agc agt aat aac ttc aac tac ggg gct tac cat tcc ctg      385
   Gln Glu Arg Ser Ser Asn Asn Phe Asn Tyr Gly Ala Tyr His Ser Leu
                   115                 120                 125
   gaa gct att tac cac gag atg gac aac att gcc gca gac ttt cct gac      433
   Glu Ala Ile Tyr His Glu Met Asp Asn Ile Ala Ala Asp Phe Pro Asp
               130                 135                 140
   ctg gcg agg agg gtg aag att gga cat tcg ttt gaa aac cgg ccg atg      481
   Leu Ala Arg Arg Val Lys Ile Gly His Ser Phe Glu Asn Arg Pro Met
           145                 150                 155
   tat gta ctg aag ttc agc act ggg aaa ggc gtg agg cgg ccg gcc gtt      529
   Tyr Val Leu Lys Phe Ser Thr Gly Lys Gly Val Arg Arg Pro Ala Val
       160                 165                 170
   tgg ctg aat gca ggc atc cat tcc cga gag tgg atc tcc cag gcc act      577
   Trp Leu Asn Ala Gly Ile His Ser Arg Glu Trp Ile Ser Gln Ala Thr
   175                 180                 185                 190
   gca atc tgg acg gca agg aag att gta tct gat tac cag agg gat cca      625
   Ala Ile Trp Thr Ala Arg Lys Ile Val Ser Asp Tyr Gln Arg Asp Pro
                   195                 200                 205
   gct atc acc tcc atc ttg gag aaa atg gat att ttc ttg ttg cct gtg      673
   Ala Ile Thr Ser Ile Leu Glu Lys Met Asp Ile Phe Leu Leu Pro Val
               210                 215                 220
   gcc aat cct gat gga tat gtg tat act caa act caa aac cga tta tgg      721
   Ala Asn Pro Asp Gly Tyr Val Tyr Thr Gln Thr Gln Asn Arg Leu Trp
           225                 230                 235
   agg aag acg cgg tcc cga aat cct gga agc tcc tgc att ggt gct gac      769
   Arg Lys Thr Arg Ser Arg Asn Pro Gly Ser Ser Cys Ile Gly Ala Asp
       240                 245                 250
   cca aat aga aac tgg aac gct agt ttt gca gga aag gga gcc agc gac      817
   Pro Asn Arg Asn Trp Asn Ala Ser Phe Ala Gly Lys Gly Ala Ser Asp
   255                 260                 265                 270
   aac cct tgc tcc gaa gtg tac cat gga ccc cac gcc aat tcg gaa gtg      865
   Asn Pro Cys Ser Glu Val Tyr His Gly Pro His Ala Asn Ser Glu Val
                   275                 280                 285
   gag gtg aaa tca gtg gta gat ttc atc caa aaa cat ggg aat ttc aag      913
   Glu Val Lys Ser Val Val Asp Phe Ile Gln Lys His Gly Asn Phe Lys
               290                 295                 300
   ggc ttc atc gac ctg cac agc tac tcg cag ctg ctg atg tat cca tat      961
   Gly Phe Ile Asp Leu His Ser Tyr Ser Gln Leu Leu Met Tyr Pro Tyr
           305                 310                 315
   ggg tac tca gtc aaa aag gcc cca gat gcc gag gaa ctc gac aag gtg     1009
   Gly Tyr Ser Val Lys Lys Ala Pro Asp Ala Glu Glu Leu Asp Lys Val
       320                 325                 330
   gcg agg ctt gcg gcc aaa gct ctg gct tct gtg tcg ggc act gag tac     1057
   Ala Arg Leu Ala Ala Lys Ala Leu Ala Ser Val Ser Gly Thr Glu Tyr
   335                 340                 345                 350
   caa gtg ggt ccc acc tgc acc act gtc tat cca gct agc ggg agc agc     1105
   Gln Val Gly Pro Thr Cys Thr Thr Val Tyr Pro Ala Ser Gly Ser Ser
                   355                 360                 365
   atc gac tgg gcg tat gac aac ggc atc aaa ttt gca ttc aca ttt gag     1153
   Ile Asp Trp Ala Tyr Asp Asn Gly Ile Lys Phe Ala Phe Thr Phe Glu
               370                 375                 380
   ttg aga gat acc ggg acc tat ggc ttc ctc ctg cca gct aac cag atc     1201
   Leu Arg Asp Thr Gly Thr Tyr Gly Phe Leu Leu Pro Ala Asn Gln Ile
           385                 390                 395
   atc ccc act gca gag gag acg tgg ctg ggg ctg aag acc atc atg gag     1249
   Ile Pro Thr Ala Glu Glu Thr Trp Leu Gly Leu Lys Thr Ile Met Glu
       400                 405                 410
   cat gtg cgg gac aac ctc tac tag gcgatggctc tgctctgtct acatttattt    1303
   His Val Arg Asp Asn Leu Tyr  *
   415                 420
   gtacccacac gtgcacgcac tgaggccatt gttaaaggag ctctttccta cctgtgtgag   1363
   tcagagccct ctgggtttgt ggagcacaca ggcctgcccc tctccagcca gctccctgga   1423
   gtcgtgtgtc ctggcggtgt ccctgcaaga actggttctg ccagcctgct caattttggt   1483
   cctgctgttt ttgatgagcc ttttgtctgt ttctccttcc accctgctgg ctgggcggct   1543
   gcactcagca tcaccccttc ctgggtggca tgtctctctc tacctcattt ttagaaccaa   1603
   agaacatctg agatgattct ctaccctcat tcacatctag ccaagccagt gacctttgct   1663
   ctggtggcac tgtgggagac accacttgtc tttaggtggg tctcaaagat gatgtagaat   1723
   ttcctttaat ttctcgcagt cttcctggaa aatattttcc tttgagcagc aaatcttgta   1783
   gggatatcag tgaaggtctc tccctccctc ctctcctgtt tttttttttt gaggcagagt   1843
   tttgctcttg ttgcccaggc tggagtgtga tgggctcgat cttggctcac cacaacctct   1903
   gcctcctggg ttcaagcaat tctcctgcct cagcctcttg agtagcttgg tttataggcg   1963
   catgccacca tgcctggcta attttgtgtt tttagtagag acagggtttc tccatgttgg   2023
   tcaggctggt ctcaaactcc caacctcagg tgatctgccc tccttggcct cccagagtgc   2083
   tgggattaca gggggagcca ctgtgccggt cccgtcccct ccttttttag gcctgaatac   2143
   aaagtagaag atcactttcc ttcactgtgc tgagaatttc tagatactac agttcttact   2203
   cctctcttcc ctttgttatt cagtgtgacc aggatgggcg ggaggggatc tgtgtcactg   2263
   taggtactgt gcccaggaag gctgggtgaa gtccccatct aaattgcagg atggcgaaat   2323
   tatccccatc tgtcctaatg ggcttccctc ctctttgcct tttgaactca cttcaaagat   2383
   gtaggcctca tcttacaggt cctaaatcac tcatctggcc tggataatct cactgccctg   2443
   gcacattccc atttgtgctg gggtatcctg tgtttccttg tcctggtttg tgtgtgtgtg   2503
   tgtgtgtgtg tgtgtgtgtg tgtgtgtttg tgtgtgtgtg tctgtctatt ttgatccggc   2563
   ccaagttcct aagtagagca agaattcatc aaccagctgc ctttgtttca tttcacctca   2623
   gcacgtacca tcgtcctttg gggggttgtt tgtttttgtt ttttgcttta accaaaatgt   2683
   ttgtaaatct taacctcctg cctaggattt gtacagcatt tggtgtgtgc ttataagcca   2743
   ataaatattc aatgtgagtt ccaaaaaaaa aaaaaaaaaa aaaaaaaaaa aa           2795
<210> 2
   <211> 421
   <212> PRT
   <213> Homo sapiens
<400> 2
   Met Arg Trp Ile Leu Phe Ile Gly Ala Leu Ile Gly Ser Ser Ile Cys
    1               5                  10                  15
   Gly Arg Glu Lys Phe Phe Gly Asp Gln Val Leu Arg Ile Asn Val Arg
               20                  25                  30
   Asn Gly Asp Glu Ile Ser Lys Leu Ser Gln Leu Val Asn Ser Asn Asn
           35                  40                  45
   Leu Lys Leu Asn Phe Trp Lys Ser Pro Ser Ser Phe Asn Arg Pro Val
       50                  55                  60
   Asp Val Leu Val Pro Ser Val Ser Leu Gln Ala Phe Lys Ser Phe Leu
   65                  70                  75                  80
   Arg Ser Gln Gly Leu Glu Tyr Ala Val Thr Ile Glu Asp Leu Gln Ala
                   85                  90                  95
   Leu Leu Asp Asn Glu Asp Asp Glu Met Gln His Asn Glu Gly Gln Glu
               100                 105                 110
   Arg Ser Ser Asn Asn Phe Asn Tyr Gly Ala Tyr His Ser Leu Glu Ala
           115                 120                 125
   Ile Tyr His Glu Met Asp Asn Ile Ala Ala Asp Phe Pro Asp Leu Ala
       130                 135                 140
   Arg Arg Val Lys Ile Gly His Ser Phe Glu Asn Arg Pro Met Tyr Val
   145                 150                 155                 160
   Leu Lys Phe Ser Thr Gly Lys Gly Val Arg Arg Pro Ala Val Trp Leu
                   165                 170                 175
   Asn Ala Gly Ile His Ser Arg Glu Trp Ile Ser Gln Ala Thr Ala Ile
               180                 185                 190
   Trp Thr Ala Arg Lys Ile Val Ser Asp Tyr Gln Arg Asp Pro Ala Ile
           195                 200                 205
   Thr Ser Ile Leu Glu Lys Met Asp Ile Phe Leu Leu Pro Val Ala Asn
       210                 215                 220
   Pro Asp Gly Tyr Val Tyr Thr Gln Thr Gln Asn Arg Leu Trp Arg Lys
   225                 230                 235                 240
   Thr Arg Ser Arg Asn Pro Gly Ser Ser Cys Ile Gly Ala Asp Pro Asn
                   245                 250                 255
   Arg Asn Trp Asn Ala Ser Phe Ala Gly Lys Gly Ala Ser Asp Asn Pro
               260                 265                 270
   Cys Ser Glu Val Tyr His Gly Pro His Ala Asn Ser Glu Val Glu Val
           275                 280                 285
   Lys Ser Val Val Asp Phe Ile Gln Lys His Gly Asn Phe Lys Gly Phe
       290                 295                 300
   Ile Asp Leu His Ser Tyr Ser Gln Leu Leu Met Tyr Pro Tyr Gly Tyr
   305                 310                 315                 320
   Ser Val Lys Lys Ala Pro Asp Ala Glu Glu Leu Asp Lys Val Ala Arg
                   325                 330                 335
   Leu Ala Ala Lys Ala Leu Ala Ser Val Ser Gly Thr Glu Tyr Gln Val
               340                 345                 350
   Gly Pro Thr Cys Thr Thr Val Tyr Pro Ala Ser Gly Ser Ser Ile Asp
           355                 360                 365
   Trp Ala Tyr Asp Asn Gly Ile Lys Phe Ala Phe Thr Phe Glu Leu Arg
       370                 375                 380
   Asp Thr Gly Thr Tyr Gly Phe Leu Leu Pro Ala Asn Gln Ile Ile Pro
   385                 390                 395                 400
   Thr Ala Glu Glu Thr Trp Leu Gly Leu Lys Thr Ile Met Glu His Val
                   405                 410                 415
   Arg Asp Asn Leu Tyr
               420
<210> 3
   <211> 1266
   <212> DNA
   <213> Homo sapiens
<400> 3
   atgaggtgga tactgttcat tggggccctt attgggtcca gcatctgtgg ccgagaaaaa     60
   ttttttgggg accaagtttt gaggattaat gtcagaaatg gagacgagat cagcaaattg     120
   agtcaactag tgaattcaaa caacttgaag ctcaatttct ggaaatctcc ctcctccttc     180
   aatcggcctg tggatgtcct ggtcccatct gtcagtctgc aggcatttaa atccttcctg     240
   agatcccagg gcttagagta cgcagtgaca attgaggacc tgcaggccct tttagacaat     300
   gaagatgatg aaatgcaaca caatgaaggg caagaacgga gcagtaataa cttcaactac     360
   ggggcttacc attccctgga agctatttac cacgagatgg acaacattgc cgcagacttt     420
   cctgacctgg cgaggagggt gaagattgga cattcgtttg aaaaccggcc gatgtatgta     480
   ctgaagttca gcactgggaa aggcgtgagg cggccggccg tttggctgaa tgcaggcatc     540
   cattcccgag agtggatctc ccaggccact gcaatctgga cggcaaggaa gattgtatct     600
   gattaccaga gggatccagc tatcacctcc atcttggaga aaatggatat tttcttgttg     660
   cctgtggcca atcctgatgg atatgtgtat actcaaactc aaaaccgatt atggaggaag     720
   acgcggtccc gaaatcctgg aagctcctgc attggtgctg acccaaatag aaactggaac     780
   gctagttttg caggaaaggg agccagcgac aacccttgct ccgaagtgta ccatggaccc     840
   cacgccaatt cggaagtgga ggtgaaatca gtggtagatt tcatccaaaa acatgggaat     900
   ttcaagggct tcatcgacct gcacagctac tcgcagctgc tgatgtatcc atatgggtac     960
   tcagtcaaaa aggccccaga tgccgaggaa ctcgacaagg tggcgaggct tgcggccaaa    1020
   gctctggctt ctgtgtcggg cactgagtac caagtgggtc ccacctgcac cactgtctat    1080
   ccagctagcg ggagcagcat cgactgggcg tatgacaacg gcatcaaatt tgcattcaca    1140
   tttgagttga gagataccgg gacctatggc ttcctcctgc cagctaacca gatcatcccc    1200
   actgcagagg agacgtggct ggggctgaag accatcatgg agcatgtgcg ggacaacctc    1260
   tactag                                                               1266

## Description

### Field of the Invention

The invention relates to methods using previously characterized carboxypeptidase polypeptides and polynucleotides as targets for the diagnosis and treatment of cellular proliferation and differentiation-related disorders.

### Background of the Invention

### Carboxypeptidases

Proteolytic enzymes are involved in many cellular processes. The carboxypeptidase family of enzymes catalyzes the cleavage of C-terminal amino acids of peptides and proteins, altering their biological activity. Lysosomal carboxypeptidase enzymes are highly concentrated in lysosomes, but may also be active extracellularly after their release from lysosomes in soluble form or bound to transmembrane or other membrane-associated proteins. Carboxypeptidases may cleave peptides in a sequence-specific manner. For example, prolylcarboxypeptidases cleave only peptides linked to proline residues (for example, des-Arg9-bradykinin, angiotensin II). There is also evidence that these enzymes are involved in terminating signal transduction by inactivating peptide ligands after receptor endocytosis.

In contrast to endoproteases which cleave internal peptide bonds of proteins and polypeptides, carboxypeptidases (CPs) catalyze the cleavage of only the C-terminal peptide bond, releasing one amino acid at a time. The two main groups of CPs include serine CPs and metallo-CPs, the serine CPs containing a signature trio of Ser, Asp, His in the active site. This trio is also contained in prolylendopeptidase serine proteases. Serine CPs include polycarboxypeptidase (PRCP) also referred to as angiotensinase C; and deamidase, also referred to as cathepsin A and lysosomal protective protein. See Skidgel et al. (1998) Immunological Reviews 161:129-141.

Metallo-CPs contain a signature glutamic acid as the primary catalytic residue and require zinc-binding for activity. Metallo-CPs can be grouped by substrate specificity into CPA and CPB types; the CPA type preferentially cleaving C-terminal hydrophobic residues, and the CPB type cleaving only peptides with C-terminal basic Arg or Lys residues. See R.A. Skidgel (1993) In: Hooper NM, ed. Zinc Metalloproteases in Health and Disease, London: Taylor & Francis, Ltd., p. 241-283.

CPM is a B type carboxypeptidase which is anchored on cell membranes via gylcosylphosphatidylinositol (GPI) association with its mildly hydrophobic stretch of 15 C-terminal amino acids. As in many other proteins sharing this anchoring mechanism, CPM is released from the membrane by bacterial phosphatidylinositol-specific phospholipase C. Human CPM is a glycoprotein of 426 amino acid residues with 43% identity to human intracellular secretory granular CP (CPE), 41% with the active 50kDa subunit of human plasma CPN, and 15% with bovine pancreatic CPA or CPB. The active sites of these CPs contain conserved amino acid residues corresponding to the zinc binding residues His66Glu69 and His173, substrate binding residues Arg137 and Tyr242, and the catalytic Glu264, as designated for CPM. Sequence homologies around these conserved residues is high, with an identity between CPs M, E and N of approximately 70-90%. See Tan et al. (1989) J. Biol. Chem. 264:13165-13170; Deddish et al. (1990) J. Biol. Chem. 265:15083-15089; R.A. Skidgel (1993) In: Hooper NM, ed. Zinc Metalloproteases in Health and Disease, London: Taylor & Francis, Ltd., p. 241-283. CPM has been mapped to the chromosomal location of chromosome 12q13-q15 which is associated with a variety of solid tumors.

The optimal pH range of CPM is in the neutral range of 6.5-7.5. As no endogenous inhibitors are known for CPM, the enzyme is considered to be constitutively active. Synthetic inhibitors including Arg analogs DL-2 mercaptomethyl-3-guanidinoethylthiopropanoic acid (MGTA) and guanidinoethylmercaptosuccinic acid (GEMSA) inhibit CPM. See R. A. Skidgel (1991) In: Conn PM, ed. Methods in Neurosciences: Peptide Technology Vol. 6, Orlando: Academic Press, p. 373-385; Plummer et al. (1981) Biochem. Biophys. Res. Comm. 98: 448-254*.*

As with other B type regulatory CPs, CPM cleaves only C-terminal Arg or Lys residues; however, CPM has a preference for the C-terminal Arg. The penultimate amino acid also affects the rate of hydrolysis. Naturally occurring peptide substrates of CPM include bradykinin, Arg6- and Lys6 enkephalins, dynorphin A1-13 and epidermal growth factor (EGF). See Sidgel et al. (1989) J. Biol. Chem. 264:2236-2241; McGwire et al. (1995) J. Biol. Chem. 270:17154-17158.

CPM is primarily found on the plasma membrane, with highest levels found in lung and placenta. It is also present in kidney, blood vessels, intestine, brain and peripheral nerves. See R.A. Skidgel (1988) Trends Pharm. Sci. 9:299-304; Skidgel et al. (1984) Biochem. Pharmacol. 33: 3471-3478; Skidgel et al. (1991) FASEB J. 5: 1578; Nagae et al. (1992) J. Neurochem. 59:2201-2212; Nagae et al. (1993) Am. J. Respir. Cell Mol. Biol. 9:221-229. Expression of CPM is responsive to differentiation of monocytes and lymphocytes. See de Saint-Vis et al. (1995) Blood 86:1098-1105; Rehli et al. (1995) J. Biol. Chem. 270:15644-15649.

CPM participates in the control of peptide hormone activity at the cell surface and degradation of extracellular proteins and peptides. It catalyzes the second step in prohormone processing and removes C-terminal Arg or Lys residues from peptides released from prohormones. CPM functions as a soluble enzyme after its release from the plasma membrane and may function in the plasma membrane form to control peptide receptor activities. CPM can regulate receptor specificity of kinins by cleaving the C-terminal ARG9, for example, from bradykinin. The intact bradykinin binds the B2 receptor. The cleaved bradykinin (des-ARG9-bradykinin). Des-ARG9-bradykinin also binds the B1 receptors: stimulates IL-1 and tumor necrosis factor release from macrophages. Regulation of the B1 receptor is associated with injury or inflammation. CPM may also be involved with other inflammatory mediators, such as anaphylatoxin C5a which mediates histamine release. In addition, CPM may metabolize growth factors containing terminal Arg or Lys, such as EGF, EGF-like peptides, nerve growth factor (NGF) amphiregulin, hepatocyte growth factor, erythropoietin, and macrophage-stimulating protein. In the lung, varying levels of CPM are associated with pneumocystic or bacterial pneumonia or lung cancer, and in the placenta, CPM may protect the fetus from maternally derived peptides. See R.A. Skidgel (1992) J. Cardiovasc. Pharmacol. 20 (Suppl. 9):S4-S9; Bhoola et al. (1992) Pharmacol. Rev. 44:1-80; R.A. Skidgel (1993) In: Hooper NM, ed. Zinc Metalloproteases in Health and Disease, London: Taylor & Francis, Ltd., p. 241-283; Dragovic et al. (1995) Am. J. Respir. Crit. Care Med. 152:760-764; Nagae et al. (1992) J. Neurochem. 59:2201-2212; MacFadden et al. (1988) FASEB J. 2:1179 (Abstract).

Another B-type regulatory CP metalloprotein is CPD, a membrane-bound glycoprotein. Human CPD is a protein of 1,377 amino acids with 75% identity with duck GP180 and 90% identity with rat CPD. Human CPD contains two hydrophobic regions located at the C- and N-termini. A 55-60 residue cytoplasmic domain is highly conserved among duck, human and rat sequences and may be significant in intracellular sorting, protein-protein interactions or endocytosis. CPD contains three tandem CP homology domains numbered sequentially from the N- to the C-terminus, and thereby may contain more than one active site. See Tan et al. (1997) Biochem. J. 327:81-87; Skidgel et al. (1993) In: Robertson JLS, Nicholls MG, eds. The Renin Angiotensin System, Vol. 1, London: Gower Medical Publishing, p. 10.1-10.10. CPD is located on human chromosome 17, 17P, 11.1-17q, 11.2.

CPD is primarily found on intracellular membranes, mainly in the Golgi, with some CPD found on the plasma membrane. The tissue distribution of CPD is wide and includes most duck tissues and mammalian tissues as well, including brain, pituitary, placenta, pancreas, adrenal, kidney, lung, heart, spleen, intestine, ovary, and testes. See McGwire et al. (1997) Life Sci. 60:715-724; Song et al. (1995) J. Biol. Chem. 270:25007-25013; Xin et al. (1997) DNA Cell Biol. 16:897-909; Tan et al. (1997) Biochem. J. 327:81-87; Song et al. (1996) J. Biol. Chem. 271:28884-28889.

The function of CPD is speculated to include peptide and protein processing in the constitutive secretory pathway after endoprotease cleavage of precursor proteins. The enzyme has an acidic pH optimum. Mammalian CPD may act as a hepatitis B virus binding protein, similar to the duck CPD. See R.A. Skidgel (1998) Immunological Reviews 161:129-141*.*

Serine CPs include PRCP and deamidase. PRCP cloned from a human kidney library indicates a glycoprotein of 51kDa3; and containing 496 amino acids, including a 30 residue signal peptide and a 15 residue propeptide. See Tan et al. (1993) J. Biol. Chem. 268:16631-16638. A serine repeat is found in the C-terminal half, similar to the serine repeat of a yeast CP encoded by the *KEX1* gene.

PRCP has an acidic pH optimum for synthetic peptide substrates, but retains activity at neutral ranges with longer naturally occurring peptides. PRCP cleaves peptides only if the penultimate residue is proline. The enzyme does not cleave Pro-Pro-COOH or (OH)-Pro-Pro-COOH bond. See Odya et al. (1978) J. Biol. Chem. 253:5927-5931. Substrates of PRCP include des-Arg9-bradykinin and angiotensin II.

PRCP may be involved in terminating signal transduction by inactivating peptide ligands after receptor endocytosis. PRCP is contained in lysosomes and released in response to stimulation. The enzyme is widely distributed and found in human placenta, lung, liver, and kidney.

Another serine CP, deamidase, is likely a 94kDa homodimer of 52kDa subunits. Human platelet deamidase is activated by cleavage of a 14 amino acid fragment from the C-terminus. The enzyme binds and maintains activity and stability of β-galactocidase and neuraminidase in lysosomes, a defect of which is associated with severe galactosialidosis. See Bonten et al. (1995) J. Biol. Chem. 270:26441-26445; Galjart et al. (1988) Cell 54:755-764; D'Azzo et al. (1982) Proc. Natl. Acad. Sci. 79:4535-4539. The gene for the human deamidase is mapped to chromosome 20 at q13.1.

Deamidase cleaves various peptides containing C-terminal or penultimate hydrophobic residues including substance P, angiotensin I, bradykinin, endothelin, and fMet-Leu-Phe. Like PRCP, deamidase is also found in lysosomes, and distributed in human placenta, lung, liver, and kidney. Like PRCP, deamidase is implicated in blocking part of the signal transduction pathway stimulated by peptides. Bradykinin, containing a C-terminal Arg9 and a penultimate hydrophobic amino acid Phe8, is cleaved by deamidase. Similarly, angiotensin, containing a C-terminal His and a penultimate Phe, is cleaved by deamidase. Accordingly, deamidase is implicated in termination of bradykinin activity on the B2 receptor to generate a B1 receptor agonist. Deamidase may also have a role in chemotaxis and in metabolism of the anti-cancer growth factor antagonist. See Skidgel et al. (1998) Immunological Reviews 161:129-141; Jackman et al. (1990) J. Biol. Chem. 265:11265-11272; Jackman et al. (1995) Am. J. Respir. Cell Mol. Biol. 13:196-204; Hinek et al. (1996) Biol. Chem. 377:471-480; Jones et al. (1995) Peptides 16:777-783; Cummings et al. (1995) Biochem Pharmacol. 49:1709-1712.

Given the wide distribution and various physiological and pathological roles of carboxypeptidases, methods and compositions directed at regulating levels of these enzymes are useful for regulating peptide hormone activity, modulating metabolism of substance P, angiotensin I, angiotensin II, bradykinin, and endothelin, and regulation of signal transduction by inactivation of peptide ligands subsequent to receptor endocytosis.

Accordingly, carboxypeptidases are a major target for drug action and development.

The carboxypeptidase gene used in the methods of the invention (GenBank Accession AF095719) was purported to be involved in the histone hyperacetylation signaling pathway relating to prostate cancer differentiation. (Huang H. et al. Cancer Res. (1999) "Carboxypeptidase A3 (CPA3): a novel gene highly induced by histone deacetylase inhibitors during differentiation of prostate epithelial cancer cells" 15;59(12):2981-8). It was suggested that the CPA3 gene is involved in the histone hyperacetylation signaling pathway activated during NaBu-mediated differentiation of the androgen-independent prostate cancer cell line, PC-3 cells.

### Summary of the Invention

17906 nucleic acid and protein molecules are useful as modulating agents in regulating a variety of cellular processes, e.g., including cell proliferation, differentiation, growth and division. In particular, 17906 nucleic acid and protein molecules will be advantageous in the regulation of any cellular function involved in uncontrolled proliferation and differentiation, such as in cases of cancer. As such, 17906 nucleic acid and protein molecules provide methods for the diagnosis and treatment of cancer preferably, breast and ovarian tumors and metastases, and most preferably breast cancer. The present invention is based, at least in part, on the discovery that the 17906 gene is up-regulated in tumor cells, and, thus, may be associated with cancer.

In one aspect the invention provided a method of identifying a subject having breast or ovarian cancer or at risk for developing breast or ovarian cancer comprising:
a) contacting a first sample obtained from the subject comprising nucleic acid molecules with a hybridization probe comprising at least 25 contiguous nucleotides of SEQ ID NO:1;
b) detecting the presence of a nucleic acid molecule in the first sample that hybridizes to the probe,
c) contacting a second sample obtained from a control subject comprising nucleic acid molecules with a hybridization probe comprising at least 25 contiguous nucleotides of SEQ ID NO:1;
d) detecting the presence of a nucleic acid molecule in the second sample that hybridizes to the probe; and
e) comparing the level of nucleic acid molecules which hybridize to the probe in the first sample with the level of nucleic acids which hybridize to the probe in the second sample,
wherein an increased level of nucleic acids which hybridize in the first sample relative to the second sample identifies a subject having breast or ovarian cancer or at risk for developing breast or ovarian, cancer.

In a further aspect, the invention provides a method of identifying a subject having breast or ovariancancer or at risk for developing breast or ovarian cancer comprising:
a) contacting a first sample obtained from the subject comprising nucleic acid molecules with a first and a second amplification primer, the first primer comprising at least 25 contiguous nucleotides of SEQ ID NO:1 and the second primer comprising at least 25 contiguous nucleotides from the complement of SEQ ID NO:1;
b) incubating the first sample under conditions that allow nucleic acid amplification;
c) contacting a second sample obtained from a control subject comprising nucleic acid molecules with a first and a second amplification primer, the first primer comprising at least 25 contiguous nucleotides of SEQ ID NO:1 and the second primer comprising at least 25 contiguous nucleotides from the complement of SEQ ID NO:1;
d) incubating the second sample under conditions that allow nucleic acid amplification; and
e) comparing the level of nucleic acid amplification in the first sample relative to the level of nucleic acid amplification in the second sample,
wherein an increased level of nucleic acid amplification in the first sample relative to the second sample identifies a subject having breast or ovarian cancer or at risk for developing breast or ovarian cancer.

In yet another aspect, the invention provides a method of identifying a subject having breast or ovarian cancer or at risk for developing breast or ovarian cancer comprising:
a) contacting a first sample obtained from the subject comprising polypeptides with an antibody which specifically binds to a polypeptide of SEQ ID NO:2;
b) detecting the presence of a polypeptide in the first sample that binds to the antibody;
c) contacting a second sample from a control subject comprising polypeptides with an antibody which specifically binds to a polypeptide of SEQ ID NO:2;
d) detecting the presence of a polypeptide in the second sample that binds to the antibody; and
e) comparing the level of antibody binding in the first sample relative to the level of antibody binding in the second sample,
wherein an increased level of antibody binding in the first sample relative to the second sample identifies a subject having breast or ovarian cancer or at risk for developing breast or ovarian cancer.

In another aspect, the invention provides a method for identifying a compound capable of treating breast or ovarian cancer characterized by increased expression of a nucleic acid molecule of SEQ ID NO:1 or increased activity of a polypeptide of SEQ ID NO:2 comprising assaying the ability of the compound to decrease expression of a nucleic acid molecule of SEQ ID NO:1 or down regulate activity of a polypeptide of SEQ ID NO:2, thereby identifying a compound capable of treating breast or ovarian cancer characterized by increased expression of a nucleic acid molecule of SEQ ID NO:1 or activity of a polypeptide of SEQ ID NO:2.

In another embodiment, the disorder is cancer. In a further embodiment, the ability of the compound to modulate the activity of the 17906 protein is determined by detecting the induction of an intracellular second messenger.

In another aspect, the invention provides a method for evaluating the efficacy of a treatment of breast or ovarian cancer, in a sample isolated from a subject, being treated with a protocol under evaluation, which comprises:
assessing the expression level of a nucleic acid molecule selected from the group consisting of:
   a) a nucleic acid molecule comprising a nucleotide sequence which is at least 80% identical to the nucleotide sequence of SEQ ID NO:1;
   b) a nucleic acid molecule which encodes a polypeptide comprising the amino acid sequence of SEQ ID NO:2; and
   c) a nucleic acid molecule comprising the nucleotide sequence of SEQ ID NO:1; and
or a the activity of a polypeptide encoded by the nucleic acid molecule,
wherein a decrease in the expression level of the nucleic acid or the polypeptide after the treatment, relative to the level before the treatment, is indicative of the efficacy of the treatment of breast or ovarian cancer.

Other features and advantages of the invention will be apparent from the following detailed description and claims.

### Brief Description of the Drawings

*Figures 1a* *and b* depicts the cDNA sequence and predicted amino acid sequence of human 17906 (GenBank Accession AF095719). The nucleotide sequence corresponds to nucleic acids 1 to 2795 of SEQ ID NO:1. The coding region corresponds to nucleic acids 8 to 1273 of SEQ ID NO:1. The amino acid sequence is identified as SEQ ID NO:2.
*Figure* 2 is a graph depicting the results of real-time quantitative RT-PCR analysis of human 17906 expression in cells related to osteoblast differentiation.
*Figure* 3 is a graph depicting the results of real-time quantitative RT-PCR analysis of human 17906 expression in various cell lines.
*Figure 4* is a graph depicting downregulation of human 17906 mRNA expression in osteoblasts treated with various compounds.
*Figure* 5 is a graph depicting downregulation of human 17906 mRNA expression in primary osteoblasts (supplied from Clonectics) that are induced with B-phosphoglycerate to differentiate.
*Figure* 6 is a breast model panel bar graph depicting the relative expression of 17906 mRNA relative to a no template control in a panel of cell lines, detected using real-time quantitative RT-PCR Taq Man analysis.
*Figure* 7 is an oncology phase II bar graph depicting the expression of 17906 mRNA relative to a no template control showing an increased expression in 4/6 breast tumors in comparison to normal breast tissues, 5/5 ovarian tumors in comparison to normal ovarian tissues, 3/7 lung tumors in comparison to normal lung tissues, and 3/4 colon tumors and 2/2 colon metastases in comparison to normal colon tissues, which expression was detected using Taq Man analysis.

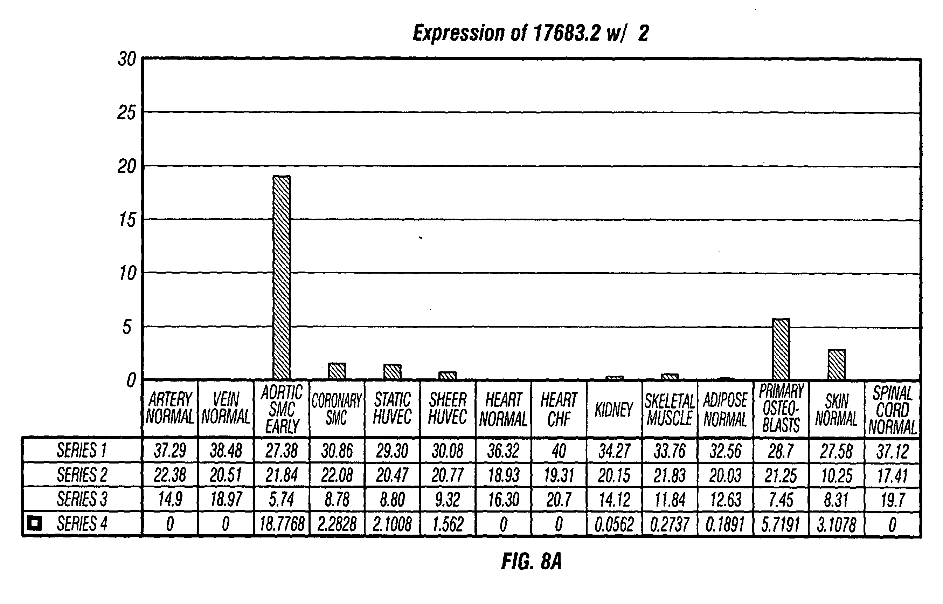

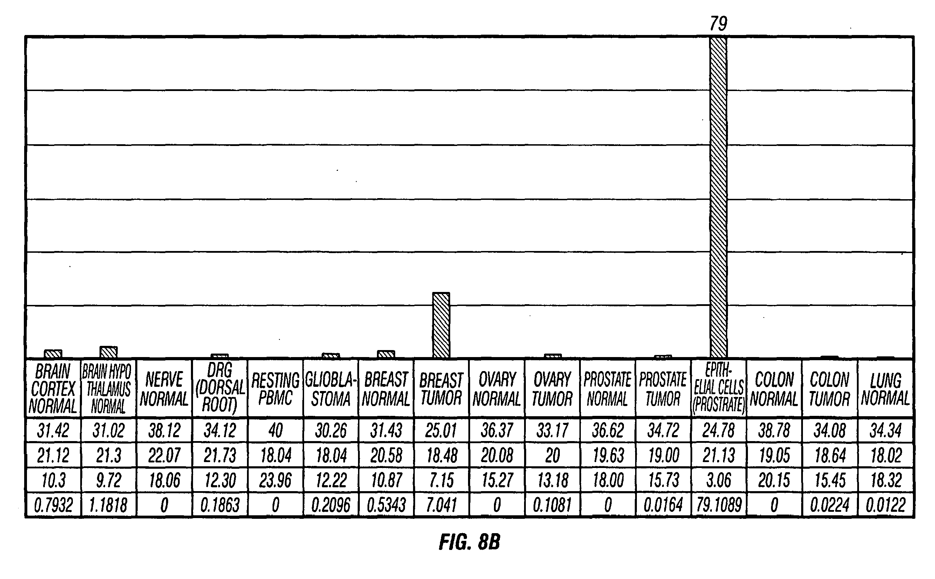

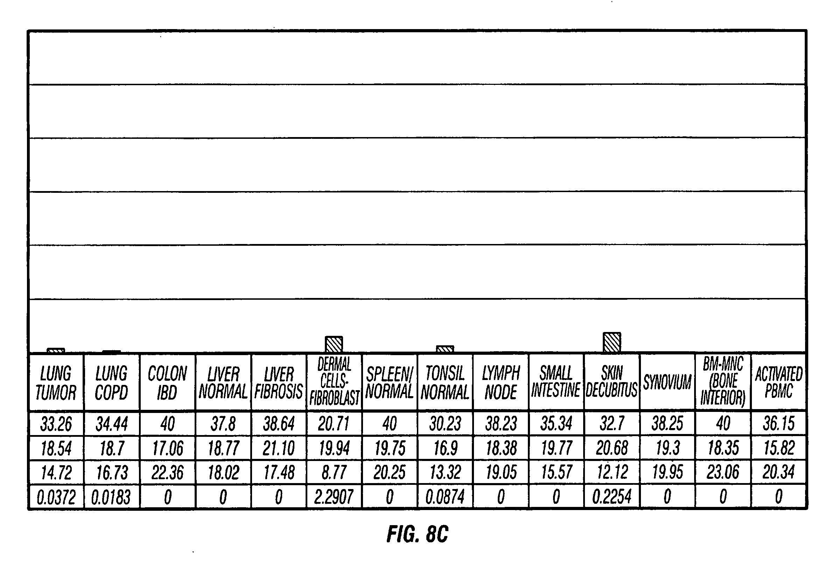

*Figure 8* is a panel bar graph depicting the relative expression of 17906 RNA relative to a no template control in a panel of human tissues or cells, including but not limited to artery, vein, aortic smooth muscle cells (SMC) (early), coronary SMC, shear and static human umbilical vein endothelial cells (HUVEC), heart, kidney, skeletal muscle, adipose, pancreas, osteoclasts, skin, spinal cord, brain cortex, brain hypothalamus, nerve dorsal root ganglia (DRS), glioblastoma, normal breast, breast tumor, normal ovary, ovary tumor, normal prostate and prostate tumor, epithelial, colon, liver, lung, fibroblasts, tonsil, bone marrow, activated PBMC, among others, detected using real-time quantitative RT-PCR Taq Man analysis. The graph indicates significant expression in prostate epithelial cells.
*Figure 9* depicts variable expression of 17906 in a xenograph panel.

### Detailed Description of the Invention

The present invention also provides methods and compositions for the diagnosis and treatment of cellular proliferative or differentiative associated disease, such as cancer particularly breast and ovarian cancer. The present invention is based, at least in part, on the discovery that carboxypeptidase genes, referred to herein as "carboxypeptidase 17906" or "17906" nucleic acid and protein molecules are up-regulated in some tumors and thus may be associated with a cellular proliferative or differentiative disorder, such as cancer.

Thus, the 17906 molecules can act as novel diagnostic targets and therapeutic agents for controlling one or more cellular proliferative and/or differentiative disorders, particularly breast and ovarian cancer.. As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, e.g., malignant tumor growth, or may be categorized as non-pathologic, i.e., a deviation from normal but not associated with a disease state, e.g., cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

As used herein, "differential expression" includes both quantitative as well as qualitative differences in the temporal and/or tissue expression pattern of a gene. Thus, a differentially expressed gene may have its expression activated or inactivated in normal versus cellular proliferative or differentiative disease conditions. The degree to which expression differs in normal versus cellular proliferative or differentiative disease or control versus experimental states need only be large enough to be visualized via standard characterization techniques, e.g., quantitative PCR, Northern analysis, or subtractive hybridization. The expression pattern of a differentially expressed gene may be used as part of a prognostic or diagnostic cellular proliferative or differentiative disease evaluation, or may be used in methods for identifying compounds useful for the treatment of cellular proliferative or differentiative disease. In addition, a differentially expressed gene involved in cellular proliferative or differentiative disease may represent a target gene such that modulation of the level of target gene expression or of target gene product activity may act to ameliorate a cellular proliferative or differentiative disease condition. Compounds that modulate target gene expression or activity of the target gene product can be used in the treatment of cellular proliferative or differentiative associated disease. Although the 17906 genes described herein may be differentially expressed with respect to cellular proliferative or differentiative associated disease, and/or their products may interact with gene products important to cellular proliferative or differentiative associated disease, the genes may also be involved in mechanisms important to additional cellular proliferative or differentiative associated processes.

The 17906 molecules may be involved in signal transduction and, thus, may function to modulate cell proliferation, differentiation, and motility. Thus, the 17906 molecules may play a role in cellular growth signaling mechanisms. As used herein, the term "cellular growth signaling mechanisms" includes signal transmission from cell receptors, e.g., G protein coupled receptors, which regulates 1) cell transversal through the cell cycle, 2) cell differentiation, 3) cell survival, and/or 4) cell migration and patterning.

Accordingly, the 17906 molecules may be involved in cellular signal transduction pathways that modulate cellular proliferation or differentiation. The 17906 molecules also may play a role or function in the transduction of signals for cell proliferation, differentiation and apoptosis. In one embodiment, the 17906 molecules modulate the activity of one or more proteins involved in cellular growth or differentiation, e.g., breast, or ovarian cell growth or differentiation.

Thus, the 17906 molecules, by participating in cellular growth signaling mechanisms, may modulate cell behavior and act as targets and therapeutic agents for controlling cellular proliferation and differentiation of cells of the breast, or ovaries.

The 17906 molecules may also act as novel diagnostic targets and therapeutic agents for cellular proliferative or differentiative diseases or disorders.

As used herein, a "cellular proliferative or differentiative disorder" or a "cellular growth related disorder" includes a disorder, disease, or condition characterized by a deregulation, e.g., an upregulation or a downregulation, of cellular growth. Cellular growth deregulation may be due to a deregulation of cellular proliferation, cell cycle progression, cellular differentiation and/or cellular hypertrophy.

The present invention provides methods for identifying the presence of a 17906 nucleic acid or polypeptide molecule associated with a cellular proliferative or differentiative disorder. In addition, the invention provides methods for identifying a subject at risk for a cellular proliferative or differentiative disorder by detecting the presence of a 17906 nucleic acid or polypeptide molecule.

The invention also provides a method for identifying a compound capable of treating a cellular proliferative or differentiative disorder characterized by aberrant 17906 nucleic acid expression or 17906 protein activity by assaying the ability of the compound to modulate the expression of a 17906 nucleic acid or the activity of a 17906 protein. Furthermore, the disclosure provides a method for treating a subject having a bone associated or cellular proliferative or differentiative disorder characterized by aberrant 17906 protein activity or aberrant 17906 nucleic acid expression by administering to the subject a 17906 modulator which is capable of modulating 17906 protein activity or 17906 nucleic acid expression.

Various aspects of the invention are described in further detail in the following subsections.

### 1. Screening Assays

The invention provides a method (also referred to herein as a "screening assay") for identifying modulators, *i.e.*, candidate or test compounds or agents (*e*.*g*., peptides, peptidomimetics, small molecules (organic or inorganic) or other drugs) which bind to 17906 proteins, have a stimulatory or inhibitory effect on, for example, 17906 expression or 17906 activity, or have a stimulatory or inhibitory effect on, for example, the expression or activity of a 17906 substrate.

These assays are designed to identify compounds that bind to a 17906 protein, bind to other cellular or extracellular proteins that interact with a 17906 protein, and interfere with the interaction of the 17906 protein with other cellular or extracellular proteins. For example, in the case of the 17906 protein, which is a transmembrane receptor-type protein, such techniques can identify ligands for such a receptor. A 17906 protein ligand can, for example, act as the basis for amelioration of cellular proliferative or differentiative diseases, such as, for example, cancer. Such compounds may include, but are not limited to peptides, antibodies, or small organic or inorganic compounds. Such compounds may also include other cellular proteins.

Compounds identified via assays such as those described herein may be useful, for example, for ameliorating cellular proliferative or differentiative disease. In instances whereby a cellular proliferative or differentiative disease condition results from an overall lower level of 17906 gene expression and/or 17906 protein in a cell or tissue, compounds that interact with the 17906 protein may include compounds which accentuate or amplify the activity of the bound 17906 protein. Such compounds would bring about an effective increase in the level of 17906 protein activity, thus ameliorating symptoms.

In other instances mutations within the 17906 gene may cause aberrant types or excessive amounts of 17906 proteins to be made which have a deleterious effect that leads to cellular proliferative or differentiative disease. Similarly, physiological conditions may cause an excessive increase in 17906 gene expression leading to cellular proliferative or differentiative disease. In such cases, compounds that bind to a 17906 protein may be identified that inhibit the activity of the 17906 protein. Assays for testing the effectiveness of compounds identified by techniques such as those described in this section are discussed herein.

In one embodiment, the invention provides assays for screening candidate or test compounds which are substrates of a 17906 protein or polypeptide or biologically active portion thereof. In another embodiment, the invention provides assays for screening candidate or test compounds which bind to or modulate the activity of a 17906 protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad Sci. 87:6378-6382); (Felici (1991) J. Mol. Biol. 222:301-310); (Ladner *supra*.).

In one embodiment, an assay is a cell-based assay in which a cell which expresses a 17906 protein or biologically active portion thereof is contacted with a test compound and the ability of the test compound to modulate 17906 activity is determined. Determining the ability of the test compound to modulate 17906 activity can be accomplished by monitoring, for example, intracellular calcium, IP3, cAMP, or diacylglycerol concentration, the phosphorylation profile of intracellular proteins, cell proliferation and/or migration, the expression of cell surface adhesion molecules, or the activity of a 17906-regulated transcription factor. The cell can be of mammalian origin, e.g., a breast, ovarian cell. In one embodiment, compounds that interact with a 17906 receptor domain can be screened for their ability to function as ligands, *i*.*e*., to bind to the 17906 receptor and modulate a signal transduction pathway. Identification of 17906 ligands, and measuring the activity of the ligand-receptor complex, leads to the identification of modulators (e.g., antagonists) of this interaction. Such modulators may be useful in the treatment of cellular proliferative or differentiative disease.

The ability of the test compound to modulate 17906 binding to a substrate or to bind to 17906 can also be determined. Determining the ability of the test compound to modulate 17906 binding to a substrate can be accomplished, for example, by coupling the 17906 substrate with a radioisotope or enzymatic label such that binding of the 17906 substrate to 17906 can be determined by detecting the labeled 17906 substrate in a complex. 17906 could also be coupled with a radioisotope or enzymatic label to monitor the ability of a test compound to modulate 17906 binding to a 17906 substrate in a complex. Determining the ability of the test compound to bind 17906 can be accomplished, for example, by coupling the compound with a radioisotope or enzymatic label such that binding of the compound to 17906 can be determined by detecting the labeled 17906 compound in a complex. For example, compounds (e.g., 17906 ligands or substrates) can be labeled with ¹²⁵I, ³⁵S, ¹⁴C, or ³H, either directly or indirectly, and the radioisotope detected by direct counting of radioemmission or by scintillation counting. Compounds can further be enzymatically labeled with, for example, horseradish peroxidase, alkaline phosphatase, or luciferase, and the enzymatic label detected by determination of conversion of an appropriate substrate to product.

The presence of 17906 in the serum of the transgenic and wild type animals can be determined by, for example, a carboxypeptidase assay. Briefly, 5 µl of serum of mice, for example, can be combined with 45 µl of 55 µM of an appropriate 17906 substrate including but not limited to e.g., angiotensin I, a kinin, or kinetensin, in 17906 buffer. Then, the rate of proteolytic degradation of the substrate can be measured by measuring the production of fluorescence (in fluorescence units) per second for 30 minutes at room temperature at a gain setting of 10. The average rate of fluorescence units per second (FU/sec) correlates directly with the amount of 17906 in the serum. As a control for the specificity of 17906, a standard carboxypeptidase assay can be performed (Holmquist and Riordan, Carboxypeptidase A, pp 44-60, Peptidase and their Inhibitors in Method of Enzymatic Analysis (1984)). Further, an additional carboxypeptidase assay can be performed in accordance with that described in Ostrowska, H. et al. (1998) Rocz Akad. Med. Bialymst., 43:39-55, which is incorporated herein by reference.

It is also within the scope of this invention to determine the ability of a compound (e.g., a 17906 ligand or substrate) to interact with 17906 without the labeling of any of the interactants. For example, a microphysiometer can be used to detect the interaction of a compound with 17906 without the labeling of either the compound or the 17906 (McConnell, H. M. et al. (1992) Science 257:1906-1912). As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 17906.

In another embodiment, an assay is a cell-based assay comprising contacting a cell expressing a 17906 target molecule (e.g., a 17906 substrate) with a test compound and determining the ability of the test compound to modulate (e.g., stimulate or inhibit) the activity of the 17906 target molecule. Determining the ability of the test compound to modulate the activity of a 17906 target molecule can be accomplished, for example, by determining the ability of the 17906 protein to bind to or interact with the 17906 target molecule.

Determining the ability of the 17906 protein or a biologically active fragment thereof, to bind to or interact with a 17906 target molecule can be accomplished by one of the methods described above for determining direct binding. In a preferred embodiment, determining the ability of the 17906 protein to bind to or interact with a 17906 target molecule can be accomplished by determining the activity of the target molecule. For example, the activity of the target molecule can be determined by detecting induction of a cellular second messenger of the target (*i*.*e*., intracellular Ca2+, diacylglycerol, IP3, cAMP), detecting catalytic/enzymatic activity of the target an appropriate substrate, detecting the induction of a reporter gene (comprising a target-responsive regulatory element operatively linked to a nucleic acid encoding a detectable marker, e.g., luciferase), or detecting a target-regulated cellular response (e.g., cell proliferation or migration).

In yet another embodiment, an assay of the present invention is a cell-free assay in which a 17906 protein or biologically active portion thereof, is contacted with a test compound and the ability of the test compound to bind to the 17906 protein or biologically active portion thereof is determined. Preferred biologically active portions of the 17906 proteins to be used in assays of the present invention include fragments which participate in interactions with non-17906 molecules, e.g., fragments with high surface probability scores. Binding of the test compound to the 17906 protein can be determined either directly or indirectly as described above. In a preferred embodiment, the assay includes contacting the 17906 protein or biologically active portion thereof with a known compound which binds 17906 to form an assay mixture, contacting the assay mixture with a test compound, and determining the ability of the test compound to interact with a 17906 protein, wherein determining the ability of the test compound to interact with a 17906 protein comprises determining the ability of the test compound to preferentially bind to 17906 or biologically active portion thereof as compared to the known compound. Compounds that modulate the interaction of 17906 with a known target protein may be useful in regulating the activity of a 17906 protein, especially a mutant 17906 protein.

In another embodiment, the assay is a cell-free assay in which a 17906 protein or biologically active portion thereof is contacted with a test compound and the ability of the test compound to modulate (e.g., stimulate or inhibit) the activity of the 17906 protein or biologically active portion thereof is determined. Determining the ability of the test compound to modulate the activity of a 17906 protein can be accomplished, for example, by determining the ability of the 17906 protein to bind to a 17906 target molecule by one of the methods described above for determining direct binding. Determining the ability of the 17906 protein to bind to a 17906 target molecule can also be accomplished using a technology such as real-time Biomolecular Interaction Analysis (BIA) (Sjolander, S. and Urbaniczky, C. (1991) Anal. Chem. 63:2338-2345 and Szabo et al. (1995) Curr. Opin. Struct. Biol. 5:699-705). As used herein, "BIA" is a technology for studying biospecific interactions in real time, without labeling any of the interactants (*e*.*g*., BIAcore). Changes in the optical phenomenon of surface plasmon resonance (SPR) can be used as an indication of real-time reactions between biological molecules.

In another embodiment, determining the ability of the test compound to modulate the activity of a 17906 protein can be accomplished by determining the ability of the 17906 protein to further modulate the activity of a downstream effector of a 17906 target molecule. For example, the activity of the effector molecule on an appropriate target can be determined or the binding of the effector to an appropriate target can be determined as previously described.

In yet another embodiment, the cell-free assay involves contacting a 17906 protein or biologically active portion thereof with a known compound which binds the 17906 protein to form an assay mixture, contacting the assay mixture with a test compound, and determining the ability of the test compound to interact with the 17906 protein, wherein determining the ability of the test compound to interact with the 17906 protein comprises determining the ability of the 17906 protein to preferentially bind to or modulate the activity of a 17906 target molecule.

In more than one embodiment of the above assay methods of the present invention, it may be desirable to immobilize either 17906 or its target molecule to facilitate separation of complexed from uncomplexed forms of one or both of the proteins, as well as to accommodate automation of the assay. Binding of a test compound to a 17906 protein, or interaction of a 17906 protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/ 17906 fusion proteins or glutathione-S-transferase/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein or 17906 protein, and the mixture incubated under conditions conducive to complex formation (*e.g*., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of 17906 binding or activity determined using standard techniques.

Other techniques for immobilizing proteins on matrices can also be used in the screening assays of the invention. For example, either a 17906 protein or a 17906 target molecule can be immobilized utilizing conjugation of biotin and streptavidin. Biotinylated 17906 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical). Alternatively, antibodies reactive with 17906 protein or target molecules but which do not interfere with binding of the 17906 protein to its target molecule can be derivatized to the wells of the plate, and unbound target or 17906 protein trapped in the wells by antibody conjugation. Methods for detecting such complexes, in addition to those described above for the GST-immobilized complexes, include immunodetection of complexes using antibodies reactive with the 17906 protein or target molecule, as well as enzyme-linked assays which rely on detecting an enzymatic activity associated with the 17906 protein or target molecule.

In another embodiment, modulators of 17906 expression are identified in a method wherein a cell is contacted with a candidate compound and the expression of 17906 mRNA or protein in the cell is determined. The level of expression of 17906 mRNA or protein in the presence of the candidate compound is compared to the level of expression of 17906 mRNA or protein in the absence of the candidate compound. The candidate compound can then be identified as a modulator of 17906 expression based on this comparison. For example, when expression of 17906 mRNA or protein is greater (statistically significantly greater) in the presence of the candidate compound than in its absence, the candidate compound is identified as a stimulator of 17906 mRNA or protein expression. Alternatively, when expression of 17906 mRNA or protein is less (statistically significantly less) in the presence of the candidate compound than in its absence, the candidate compound is identified as an inhibitor of 17906 mRNA or protein expression. The level of 17906 mRNA or protein expression in the cells can be determined by methods described herein for detecting 17906 mRNA or protein.

In yet another aspect of the invention, the 17906 proteins can be used as "bait proteins" in a two-hybrid assay or three-hybrid assay (see, *e*.*g*., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with 17906 ("17906-binding proteins" or "17906-bp") and are involved in 17906 activity. Such 17906-binding proteins are also likely to be involved in the propagation of signals by the 17906 proteins or 17906 targets as, for example, downstream elements of a 17906-mediated signaling pathway. Alternatively, such 17906-binding proteins are likely to be 17906 inhibitors.

The two-hybrid system is based on the modular nature of most transcription factors, which consist of separable DNA-binding and activation domains. Briefly, the assay utilizes two different DNA constructs. In one construct, the gene that codes for a 17906 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a 17906-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 17906 protein.

In another aspect, the invention pertains to a combination of two or more of the assays described herein. For example, a modulating agent can be identified using a cell-based or a cell free assay, and the ability of the agent to modulate the activity of a 17906 protein can be confirmed *in vivo*, *e*.*g*., in an animal such as an animal model for cellular proliferative or differentiative disease, as described herein.

This disclosure further pertains to novel agents identified by the above-described screening assays. Accordingly, it is within the scope of this disclosure to further use an agent identified as described herein in an appropriate animal model. For example, an agent identified as described herein (e.g., a 17906 modulating agent, an antisense 17906 nucleic acid molecule, a 17906-specific antibody, or a 17906-binding partner) can be used in an animal model to determine the efficacy, toxicity, or side effects of treatment with such an agent. Alternatively, an agent identified as described herein can be used in an animal model to determine the mechanism of action of such an agent. Furthermore, this disclosure pertains to uses of novel agents identified by the above-described screening assays for treatments as described herein.

Any of the compounds, including but not limited to compounds such as those identified in the foregoing assay systems, may be tested for the ability to ameliorate bone associated or cellular proliferative or differentiative disease symptoms. Cell-based and animal model-based assays for the identification of compounds exhibiting such an ability to ameliorate bone associated or cellular proliferative or differentiative disease systems are described herein.

In one aspect, cell-based systems, as described herein, may be used to identify compounds which may act to ameliorate cellular proliferative or differentiative disease symptoms. For example, such cell systems may be exposed to a compound, suspected of exhibiting an ability to ameliorate cellular proliferative or differentiative disease symptoms, at a sufficient concentration and for a time sufficient to elicit such an amelioration of cellular proliferative or differentiative disease symptoms in the exposed cells. After exposure, the cells are examined to determine whether one or more of the cellular proliferative or differentiative disease cellular phenotypes has been altered to resemble a more normal or more wild type phenotype. Cellular phenotypes that are associated with cellular proliferative or differentiative disease states include aberrant proliferation and migration, deposition of extracellular matrix components, and expression of growth factors, cytokines, and other inflammatory mediators.

In addition, animal-based cellular proliferative or differentiative disease systems, such as those described herein, may be used to identify compounds capable of ameliorating cellular proliferative or differentiative disease symptoms. Such animal models may be used as test substrates for the identification of drugs, pharmaceuticals, therapies, and interventions which may be effective in treating cellular proliferative or differentiative disease. For example, animal models may be exposed to a compound, suspected of exhibiting an ability to ameliorate cellular proliferative or differentiative disease symptoms, at a sufficient concentration and for a time sufficient to elicit such an amelioration of cellular proliferative or differentiative disease symptoms in the exposed animals. The response of the animals to the exposure may be monitored by assessing the reversal of disorders associated with cellular proliferative or differentiative disease.With regard to intervention, any treatments which reverse any aspect of cellular proliferative or differentiative disease symptoms should be considered as candidates for human cellular proliferative or differentiative disease therapeutic intervention. Dosages of test agents may be determined by deriving dose-response curves.

Additionally, gene expression patterns may be utilized to assess the ability of a compound to ameliorate cellular proliferative or differentiative disease symptoms. For example, the expression pattern of one or more genes may form part of a "gene expression profile" or "transcriptional profile" which may be then be used in such an assessment. "Gene expression profile" or "transcriptional profile", as used herein, includes the pattern of mRNA expression obtained for a given tissue or cell type under a given set of conditions. Such conditions may include, but are not limited to, atherosclerosis, ischemia/reperfusion, hypertension, restenosis, and arterial inflammation, including any of the control or experimental conditions described herein. Gene expression profiles may be generated, for example, by utilizing a differential display procedure, Northern analysis and/or real-time quantitative RT-PCR. In one embodiment, 17906 gene sequences may be used as probes and/or PCR primers for the generation and corroboration of such gene expression profiles.

Gene expression profiles may be characterized for known states, either cellular proliferative or differentiative disease or normal, within the cell- and/or animal-based model systems. Subsequently, these known gene expression profiles may be compared to ascertain the effect a test compound has to modify such gene expression profiles, and to cause the profile to more closely resemble that of a more desirable profile.

For example, administration of a compound may cause the gene expression profile of a cellular proliferative or differentiative disease model system to more closely resemble the control system. Administration of a compound may, alternatively, cause the gene expression profile of a control system to begin to mimic a cellular proliferative or differentiative disease state. Such a compound may, for example, be used in further characterizing the compound of interest, or may be used in the generation of additional animal models.

### 2. Predictive Medicine

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining 17906 protein and/or nucleic acid expression as well as 17906 activity, in the context of a biological sample (e.g., blood, serum, cells, tissue) to thereby determine whether an individual is afflicted with a disease or disorder, or is at risk of developing a cellular proliferative or differentiative disorder, associated with aberrant or unwanted 17906 expression or activity. The invention also provides for prognostic (or predictive) assays for determining whether an individual is at risk of developing a disorder associated with 17906 protein, nucleic acid expression or activity. For example, mutations in a 17906 gene can be assayed in a biological sample. Such assays can be used for prognostic or predictive purpose to thereby prophylactically treat an individual prior to the onset of a disorder characterized by or associated with 17906 protein, nucleic acid expression or activity.

Another aspect of the invention pertains to monitoring the influence of agents (e.g., drugs, compounds) on the expression or activity of 17906 in clinical trials.

These and other agents are described in further detail in the following sections.

### A. Diagnostic Assays

The present invention encompasses methods for diagnostic and prognostic evaluation of cellular proliferative or differentiative disease conditions, and for the identification of subjects exhibiting a predisposition to such conditions.

An exemplary method for detecting the presence or absence of 17906 protein or nucleic acid in a biological sample involves obtaining a biological sample from a test subject and contacting the biological sample with a compound or an agent capable of detecting 17906 protein or nucleic acid (e.g., mRNA, or genomic DNA) that encodes 17906 protein such that the presence of 17906 protein or nucleic acid is detected in the biological sample. A preferred agent for detecting 17906 mRNA or genomic DNA is a labeled nucleic acid probe capable of hybridizing to 17906 mRNA or genomic DNA. The nucleic acid probe can be, for example, the 17906 nucleic acid set forth in SEQ ID NO:1, or a portion thereof, such as an oligonucleotide of at least 15, 20, 25, 30, 35, 40, 45, 50, 100, 250 or 500 nucleotides in length and sufficient to specifically hybridize under stringent conditions to 17906 mRNA or genomic DNA. Other suitable probes for use in the diagnostic assays of the invention are described herein.

A preferred agent for detecting 17906 protein is an antibody capable of binding to 17906 protein, preferably an antibody with a detectable label. Antibodies can be polyclonal, or more preferably, monoclonal. An intact antibody, or a fragment thereof (e.g., Fab or F(ab')2) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect 17906 mRNA, protein, or genomic DNA in a biological sample *in vitro* as well as *in vivo.* For example, *in vitro* techniques for detection of 17906 mRNA include Northern hybridizations and *in situ* hybridizations. *In vitro* techniques for detection of 17906 protein include enzyme linked immunosorbent assays (ELISAs), Western blots, immunoprecipitations and immunofluorescence. *In vitro* techniques for detection of 17906 genomic DNA include Southern hybridizations. Furthermore, *in vivo* techniques for detection of 17906 protein include introducing into a subject a labeled anti-17906 antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting 17906 protein, mRNA, or genomic DNA, such that the presence of 17906 protein, mRNA or genomic DNA is detected in the biological sample, and comparing the presence of 17906 protein, mRNA or genomic DNA in the control sample with the presence of 17906 protein, mRNA or genomic DNA in the test sample.

The disclosure also encompasses kits for detecting the presence of 17906 in a biological sample. For example, the kit can comprise a labeled compound or agent capable of detecting 17906 protein or mRNA in a biological sample; means for determining the amount of 17906 in the sample; and means for comparing the amount of 17906 in the sample with a standard. The compound or agent can be packaged in a suitable container. The kit can further comprise instructions for using the kit to detect 17906 protein or nucleic acid.

### B. Prognostic Assays

The diagnostic methods described herein can furthermore be utilized to identify subjects having or at risk of developing a cellular proliferative or differentiative disease or disorder associated with aberrant or unwanted 17906 expression or activity. As used herein, the term "aberrant" includes a 17906 expression or activity which deviates from the wild type 17906 expression or activity. Aberrant expression or activity includes increased or decreased expression or activity, as well as expression or activity which does not follow the wild type developmental pattern of expression or the subcellular pattern of expression. For example, aberrant 17906 expression or activity is intended to include the cases in which a mutation in the 17906 gene causes the 17906 gene to be under-expressed or over-expressed and situations in which such mutations result in a non-functional 17906 protein or a protein which does not function in a wild-type fashion, e.g., a protein which does not interact with a 17906 ligand or substrate, or one which interacts with a non-17906 ligand or substrate. As used herein, the term "unwanted" includes an unwanted phenomenon involved in a biological response such as cellular proliferation. For example, the term unwanted includes a 17906 expression pattern or a 17906 protein activity which is undesirable in a subject.

The assays described herein, such as the preceding diagnostic assays or the following assays, can be utilized to identify a subject having or at risk of developing a disorder associated with a misregulation in 17906 protein activity or nucleic acid expression, such as a cellular proliferative or differentiative disorder. Alternatively, the prognostic assays can be utilized to identify a subject having or at risk for developing a cellular proliferative or differentiative disorder associated with a misregulation in 17906 protein activity or nucleic acid expression. Thus, the present invention provides a method for identifying a disease or disorder associated with aberrant or unwanted 17906 expression or activity in which a test sample is obtained from a subject and 17906 protein or nucleic acid (*e*.*g*., mRNA or genomic DNA) is detected, wherein the presence of 17906 protein or nucleic acid is diagnostic for a subject having or at risk of developing a disease or disorder associated with aberrant or unwanted 17906 expression or activity. As used herein, a "test sample" refers to a biological sample obtained from a subject of interest. For example, a test sample can be a biological fluid (*e*.*g*., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 17906 expression or activity. For example, such methods can be used to determine whether a subject can be effectively treated with an agent for a cellular proliferative or differentiative disorder. Thus, the present invention provides methods for determining whether a subject can be effectively treated with an agent for cellular proliferative or differentiative disorder associated with aberrant or unwanted 17906 expression or activity in which a test sample is obtained and 17906 protein or nucleic acid expression or activity is detected (e.g., wherein the abundance of 17906 protein or nucleic acid expression or activity is diagnostic for a subject that can be administered the agent to treat a disorder associated with aberrant or unwanted 17906 expression or activity).

The methods of the disclosure can also be used to detect genetic alterations in a 17906 gene, thereby determining if a subject with the altered gene is at risk for a disorder characterized by misregulation in 17906 protein activity or nucleic acid expression, such as a proliferative disorder. In preferred embodiments, the methods include detecting, in a sample of cells from the subject, the presence or absence of a genetic alteration characterized by at least one of an alteration affecting the integrity of a gene encoding a 17906-protein, or the mis-expression of the 17906 gene. For example, such genetic alterations can be detected by ascertaining the existence of at least one of 1) a deletion of one or more nucleotides from a 17906 gene; 2) an addition of one or more nucleotides to a 17906 gene; 3) a substitution of one or more nucleotides of a 17906 gene, 4) a chromosomal rearrangement of a 17906 gene; 5) an alteration in the level of a messenger RNA transcript of a 17906 gene, 6) aberrant modification of a 17906 gene, such as of the methylation pattern of the genomic DNA, 7) the presence of a non-wild type splicing pattern of a messenger RNA transcript of a 17906 gene, 8) a non-wild type level of a 17906-protein, 9) allelic loss of a 17906 gene, and 10) inappropriate post-translational modification of a 17906-protein. As described herein, there are a large number of assays known in the art which can be used for detecting alterations in a 17906 gene. A preferred biological sample is a tissue or serum sample isolated by conventional means from a subject.

In certain embodiments, detection of the alteration involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, *e*.*g*., Landegran et al. (1988) Science 241:1077-1080; and Nakazawa et al. (1994) Proc. Natl. Acad. Sci. USA 91:360-364), the latter of which can be particularly useful for detecting point mutations in the 17906-gene (see Abravaya et al. (1995) Nucleic Acids Res .23:675-682). This method can include the steps of collecting a sample of cells from a subject, isolating nucleic acid (*e*.*g*., genomic, mRNA or both) from the cells of the sample, contacting the nucleic acid sample with one or more primers which specifically hybridize to a 17906 gene under conditions such that hybridization and amplification of the 17906-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Other amplification methods include: self sustained sequence replication (Guatelli, J.C. et al., (1990) Proc. Natl. Acad. Sci. USA 87:1874-1878), transcriptional amplification system (Kwoh, D.Y. et al., (1989) Proc. Natl. Acad. Sci. USA 86:1173-1177), Q-Beta Replicase (Lizardi, P.M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a 17906 gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 17906 can be identified by hybridizing a sample and control nucleic acids, *e.g.,* DNA or RNA, to high density arrays containing hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in 17906 can be identified in two dimensional arrays containing light-generated DNA probes as described in Cronin, M.T. *et al*. *supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 17906 gene and detect mutations by comparing the sequence of the sample 17906 with the corresponding wild-type (control) sequence. Examples of sequencing reactions include those based on techniques developed by Maxam and Gilbert ((1977) Proc. Natl. Acad Sci. USA 74:560) or Sanger ((1977) Proc. Natl. Acad. Sci. USA 74:5463). It is also contemplated that any of a variety of automated sequencing procedures can be utilized when performing the diagnostic assays ((1995) *Biotechniques* 19:448), including sequencing by mass spectrometry (see, *e*.*g*., PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in the 17906 gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type 17906 sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in 17906 cDNAs obtained from samples of cells. For example, the mutY enzyme of E. *coli* cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a 17906 sequence, *e*.*g*., a wild-type 17906 sequence, is hybridized to a cDNA or other DNA product from a test cell(s). The duplex is treated with a DNA mismatch repair enzyme, and the cleavage products, if any, can be detected from electrophoresis protocols or the like (described in, for example, U.S. Patent No. 5,459,039).

In other embodiments, alterations in electrophoretic mobility will be used to identify mutations in 17906 genes. For example, single strand conformation polymorphism (SSCP) may be used to detect differences in electrophoretic mobility between mutant and wild type nucleic acids (Orita et al. (1989) Proc Natl. Acad. Sci USA: 86:2766, see also Cotton (1993) Mutat. Res. 285:125-144; and Hayashi (1992) Genet. Anal. Tech. Appl. 9:73-79). Single-stranded DNA fragments of sample and control 17906 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl Acad Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving a 17906 gene.

Furthermore, any cell type or tissue in which 17906 is expressed may be utilized in the prognostic assays described herein.

### C. Monitoring of Effects During Clinical Trials

The present invention provides methods for evaluating the efficacy of drugs and monitoring the progress of patients involved in clinical trials for the treatment of cellular proliferative or differentiative disease.

Monitoring the influence of agents (*e*.*g*., drugs) on the expression or activity of a 17906 protein (*e*.*g*., the modulation of cell proliferation and/or migration) can be applied not only in basic drug screening, but also in clinical trials. For example, the effectiveness of an agent determined by a screening assay as described herein to increase 17906 gene expression, protein levels, or upregulate 17906 activity, can be monitored in clinical trials of subjects exhibiting decreased 17906 gene expression, protein levels, or downregulated 17906 activity. Alternatively, the effectiveness of an agent determined by a screening assay to decrease 17906 gene expression, protein levels, or downregulate 17906 activity, can be monitored in clinical trials of subjects exhibiting increased 17906 gene expression, protein levels, or upregulated 17906 activity. In such clinical trials, the expression or activity of a 17906 gene, and preferably, other genes that have been implicated in, for example, a 17906-associated disorder can be used as a "read out" or markers of the phenotype a particular cell, *e*.*g*., breast, ovary cell. In addition, the expression of a 17906 gene, or the level of 17906 protein activity may be used as a read out of a particular drug or agent's effect on a cellular proliferative or differentiative disease state.

For example, and not by way of limitation, genes, including 17906, that are modulated in cells by treatment with an agent (*e*.*g*., compound, drug or small molecule) which modulates 17906 activity (*e*.*g*., identified in a screening assay as described herein) can be identified. Thus, to study the effect of agents on 17906-associated disorders (*e*.*g*., cellular proliferative or differentiative disorders characterized by deregulated breast, ovary cell activity), for example, in a clinical trial, cells can be isolated and RNA prepared and analyzed for the levels of expression of 17906 and other genes implicated in the 17906-associated disorder, respectively. The levels of gene expression (*e*.*g*., a gene expression pattern) can be quantified by northern blot analysis or real-time quantitative RT-PCR, as described herein, or alternatively by measuring the amount of protein produced, by one of the methods as described herein, or by measuring the levels of activity of 17906 or other genes. In this way, the gene expression pattern can serve as a marker, indicative of the physiological response of the cells to the agent. Accordingly, this response state may be determined before, and at various points during treatment of the individual with the agent.

In a preferred embodiment, the present invention provides a method for monitoring the effectiveness of treatment of a subject with an agent (*e*.*g*., an agonist, antagonist, peptidomimetic, protein, peptide, nucleic acid, small molecule, or other drug candidate identified by the screening assays described herein) including the steps of (i) obtaining a pre-administration sample from a subject prior to administration of the agent; (ii) detecting the level of expression of a 17906 protein, mRNA, or genomic DNA in the preadministration sample; (iii) obtaining one or more post-administration samples from the subject; (iv) detecting the level of expression or activity of the 17906 protein, mRNA, or genomic DNA in the post-administration samples; (v) comparing the level of expression or activity of the 17906 protein, mRNA, or genomic DNA in the pre-administration sample with the 17906 protein, mRNA, or genomic DNA in the post administration sample or samples; and (vi) altering the administration of the agent to the subject accordingly. For example, increased administration of the agent may be desirable to increase the expression or activity of 17906 to higher levels than detected, *i*.*e*., to increase the effectiveness of the agent. Alternatively, decreased administration of the agent may be desirable to decrease expression or activity of 17906 to lower levels than detected, *i*.*e*. to decrease the effectiveness of the agent. According to such an embodiment, 17906 expression or activity may be used as an indicator of the effectiveness of an agent, even in the absence of an observable phenotypic response.

### Isolated Nucleic Acid Molecules

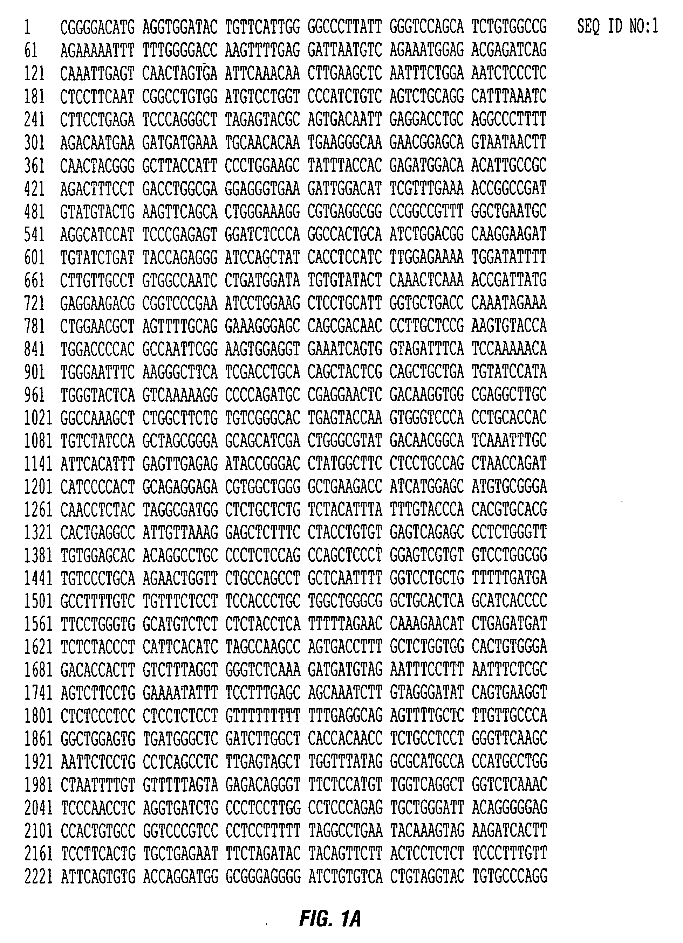

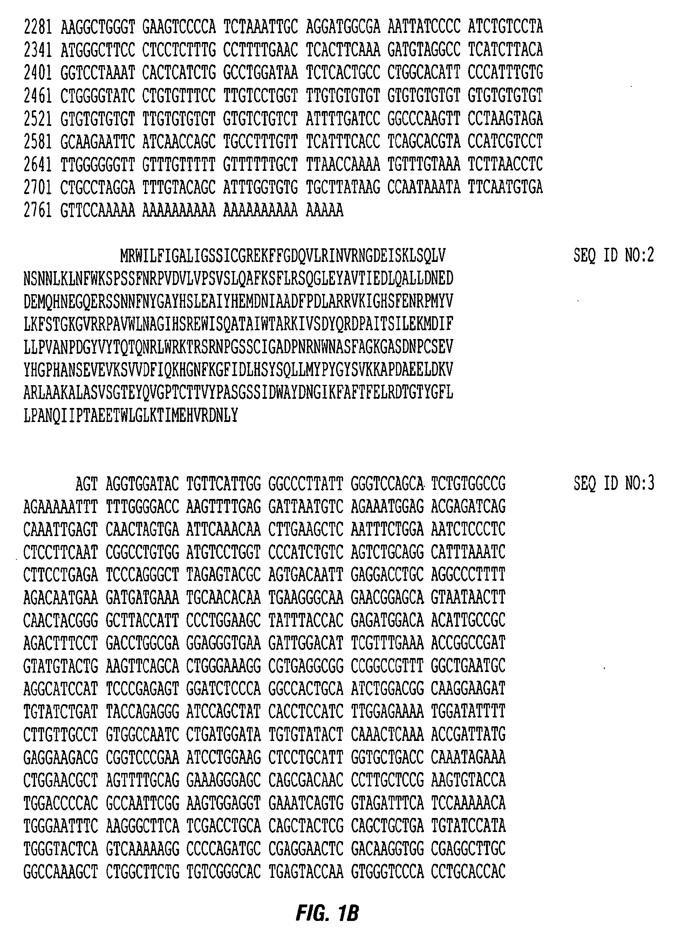

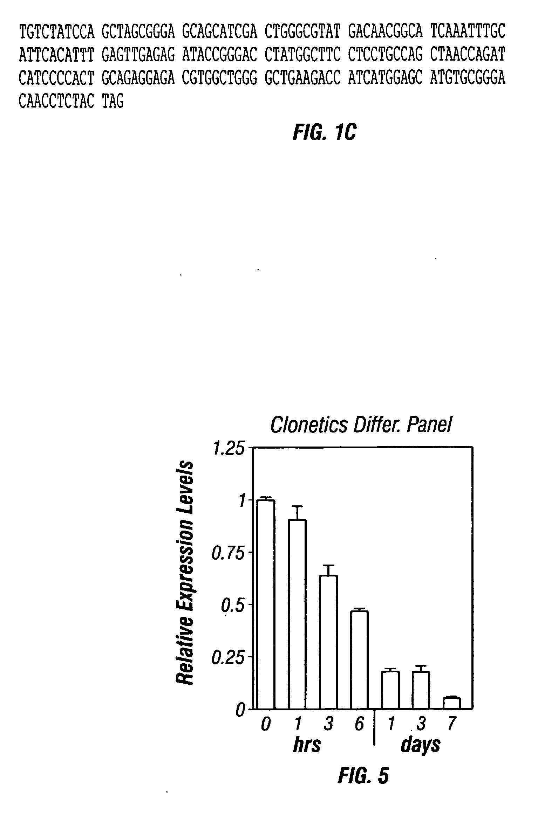

The nucleotide sequence of the isolated human 17906 cDNA and the predicted amino acid sequence of the human 17906 polypeptide are shown in Figure 1 and in SEQ ID NOs:1 and 2, respectively. The nucleotide sequence encoding human 17906 is identical to the nucleic acid molecule with GenBank Accession Number AF095719 (Huang, H. et al. Cancer Res. (1999) 59(12):2981-2988).

The human 17906 gene, which is approximately 2795 nucleotides in length, encodes a protein having a molecular weight of approximately 46.4 kD and which is approximately 422 amino acid residues in length.

The methods of the invention include the use of isolated nucleic acid molecules that encode 17906 proteins or biologically active portions thereof, as well as nucleic acid fragments sufficient for use as hybridization probes to identify 17906-encoding nucleic acid molecules (*e*.*g*., 17906 mRNA) and fragments for use as PCR primers for the amplification or mutation of 17906 nucleic acid molecules. As used herein, the term "nucleic acid molecule" is intended to include DNA molecules (e.g., cDNA or genomic DNA) and RNA molecules (*e*.*g*., mRNA) and analogs of the DNA or RNA generated using nucleotide analogs. The nucleic acid molecule can be single-stranded or double-stranded, but preferably is double-stranded DNA.

The term "isolated nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (*i*.*e*., sequences located at the 5' and 3' ends of the nucleic acid) in the genomic DNA of the organism from which the nucleic acid is derived. For example, in various embodiments, the isolated 17906 nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present disclosure, *e*.*g*., a nucleic acid molecule having the nucleotide sequence of SEQ ID NO:1, or a portion thereof, can be isolated using standard molecular biology techniques and the sequence information provided herein. Using all or portion of the nucleic acid sequence of SEQ ID NO:1, as a hybridization probe, 17906 nucleic acid molecules can be isolated using standard hybridization and cloning techniques (e.g., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989).

Moreover, a nucleic acid molecule encompassing all or a portion of SEQ ID NO:1 can be isolated by the polymerase chain reaction (PCR) using synthetic oligonucleotide primers designed based upon the sequence of SEQ ID NO:1.

A nucleic acid of the disclosure can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to 17906 nucleotide sequences can be prepared by standard synthetic techniques, e.g., using an automated DNA synthesizer.

In a preferred embodiment, an isolated nucleic acid molecule of the disclosure comprises the nucleotide sequence shown in SEQ ID NO:1. The sequence of SEQ ID NO:1 corresponds to the human 17906 cDNA. This cDNA comprises sequences encoding the human 17906 protein (*i*.*e*., "the coding region of SEQ ID NO:1").

In another preferred embodiment, an isolated nucleic acid molecule of the disclosure comprises a nucleic acid molecule which is a complement of the nucleotide sequence shown in SEQ ID NO:1, or a portion of any of this nucleotide sequence. A nucleic acid molecule which is complementary to the nucleotide sequence shown in SEQ ID NO:1 is one which is sufficiently complementary to the nucleotide sequence shown in SEQ ID NO:1 such that it can hybridize to the nucleotide sequence shown in SEQ ID NO:1, thereby forming a stable duplex.

In still another preferred embodiment, an isolated nucleic acid molecule of the present disclosure comprises a nucleotide sequence which is at least about 30%, 35%, 40%, 45%, 50%, 55%, 60%, 65%, 70%, 75%, 80%, 85%, 90%, 95%, 98% or more identical to the entire length of the nucleotide sequence shown in SEQ ID NO:1, or a portion of any of this nucleotide sequence.

Moreover, the nucleic acid molecule of the disclosure can comprise only a portion of the nucleic acid sequence of SEQ ID NO:1, for example, a fragment which can be used as a probe or primer or a fragment encoding a portion of a 17906 protein, *e*.*g*., a biologically active portion of a 17906 protein. The nucleotide sequence determined from the cloning of the 17906 gene allows for the generation of probes and primers designed for use in identifying and/or cloning other 17906 family members, as well as 17906 homologues from other species. The probe/primer typically comprises substantially purified oligonucleotide. The oligonucleotide typically comprises a region of nucleotide sequence that hybridizes under stringent conditions to at least about 12 or 15, preferably about 20 or 25, more preferably about 30, 35, 40, 45, 50, 55, 60, 65, or 75 consecutive nucleotides of a sense sequence of SEQ ID NO:1, of an anti-sense sequence of SEQ ID NO:1, or of a naturally occurring allelic variant or mutant of SEQ ID NO:1. In one embodiment, a nucleic acid molecule of the present disclosure comprises a nucleotide sequence which is greater than 100, 100-200, 200-300, 300-400, 400-500, 500-600, 600-700, 700-800, or more nucleotides in length and hybridizes under stringent hybridization conditions to a nucleic acid molecule of SEQ ID NO:1.

Probes based on the 17906 nucleotide sequence can be used to detect transcripts or genomic sequences encoding the same or homologous proteins. In preferred embodiments, the probe further comprises a label group attached thereto, *e*.*g*., the label group can be a radioisotope, a fluorescent compound, an enzyme, or an enzyme co-factor. Such probes can be used as a part of a diagnostic test kit for identifying cells or tissue which misexpress a 17906 protein, such as by measuring a level of a 17906-encoding nucleic acid in a sample of cells from a subject *e*.*g*., detecting 17906 mRNA levels or determining whether a genomic 17906 gene has been mutated or deleted.

A nucleic acid fragment encoding a "biologically active portion of a 17906 protein" can be prepared by isolating a portion of the nucleotide sequence of SEQ ID NO:1 which encodes a polypeptide having a 17906 biological activity (the biological activities of the 17906 protein is described herein), expressing the encoded portion of the 17906 protein (*e*.*g*., by recombinant expression *in vitro*) and assessing the activity of the encoded portion of the 17906 protein.

The methods of the invention further encompass nucleic acid molecules that differ from the nucleotide sequence shown in SEQ ID NO:1, due to degeneracy of the genetic code and thus encode the same 17906 protein as those encoded by the nucleotide sequence shown in SEQ ID NO:1. In another embodiment, an isolated nucleic acid molecule of the disclosure has a nucleotide sequence encoding a protein having an amino acid sequence shown in SEQ ID NO:2.

In addition to the 17906 nucleotide sequence shown in SEQ ID NO:1, it will be appreciated by those skilled in the art that DNA sequence polymorphisms that lead to changes in the amino acid sequences of the 17906 protein may exist within a population (*e*.*g*., the human population). Such genetic polymorphism in the 17906 gene may exist among individuals within a population due to natural allelic variation. As used herein, the terms "gene" and "recombinant gene" refer to nucleic acid molecules which include an open reading frame encoding a 17906 protein, preferably a mammalian 17906 protein, and can further include non-coding regulatory sequences, and introns.

Allelic variants of human 17906 include both functional and non-functional 17906 proteins. Functional allelic variants are naturally occurring amino acid sequence variants of the human 17906 protein that maintain the ability to bind a 17906 ligand or substrate and/or modulate cell proliferation and/or migration mechanisms. Functional allelic variants will typically contain only conservative substitution of one or more amino acids of SEQ ID NO:2, or substitution, deletion or insertion of non-critical residues in non-critical regions of the protein.

Non-functional allelic variants are naturally occurring amino acid sequence variants of the human 17906 protein that do not have the ability to either bind a 17906 ligand or substrate and/or modulate cell proliferation and/or migration mechanisms. Non-functional allelic variants will typically contain a non-conservative substitution, a deletion, or insertion or premature truncation of the amino acid sequence of SEQ ID NO:2, or a substitution, insertion or deletion in critical residues or critical regions.

The methods of the present invention may further use non-human orthologues of the human 17906 protein. Orthologues of the human 17906 protein are proteins that are isolated from non-human organisms and possess the same 17906 ligand binding and/or modulation of cell proliferation and/or migration mechanisms of the human 17906 protein. Orthologues of the human 17906 protein can readily be identified as comprising an amino acid sequence that is substantially identical to SEQ ID NO:2.

Moreover, nucleic acid molecules encoding other 17906 family members and, thus, which have a nucleotide sequence which differs from the 17906 sequence of SEQ ID NO:1 are intended to be within the scope of the disclosure. For example, another 17906 cDNA can be identified based on the nucleotide sequence of human 17906. Moreover, nucleic acid molecules encoding 17906 proteins from different species, and which, thus, have a nucleotide sequence which differs from the 17906 sequence of SEQ ID NO:1 are intended to be within the scope of the disclosure. For example, a mouse 17906 cDNA can be identified based on the nucleotide sequence of human 17906.

Nucleic acid molecules corresponding to natural allelic variants and homologues of the 17906 cDNA of the disclosure can be isolated based on their homology to the 17906 nucleic acid disclosed herein using the cDNAs disclosed herein, or a portion thereof, as a hybridization probe according to standard hybridization techniques under stringent hybridization conditions. Nucleic acid molecules corresponding to natural allelic variants and homologues of the 17906 cDNA of the disclosure can further be isolated by mapping to the same chromosome or locus as the 17906 gene.

Accordingly, in another embodiment, an isolated nucleic acid molecule of the disclosure is at least 15, 20, 25, 30 or more nucleotides in length and hybridizes under stringent conditions to the nucleic acid molecule comprising the nucleotide sequence of SEQ ID NO:1. In other embodiment, the nucleic acid is at least 30, 50, 100, 150, 200, 250, 300, 350, 400, 450, 500, 550, 600, 650, 700, 750, 800, 1000, 1200, or more nucleotides in length. As used herein, the term "hybridizes under stringent conditions" is intended to describe conditions for hybridization and washing under which nucleotide sequences at least 60% identical to each other typically remain hybridized to each other. Preferably, the conditions are such that sequences at least about 70%, more preferably at least about 80%, even more preferably at least about 85% or 90% identical to each other typically remain hybridized to each other. Such stringent conditions are known to those skilled in the art and can be found in *Current Protocols in Molecular Biology*, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50°C, preferably at 55°C, more preferably at 60°C, and even more preferably at 65°C. Ranges intermediate to the above-recited values, e.g., at 60-65 °C or at 55-60 °C are also intended to be encompassed by the present disclosure. Preferably, an isolated nucleic acid molecule of the disclosure that hybridizes under stringent conditions to the sequence of SEQ ID NO:1 corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally-occurring allelic variants of the 17906 sequences that may exist in the population, the skilled artisan will further appreciate that changes can be introduced by mutation into the nucleotide sequence of SEQ ID NO:1, thereby leading to changes in the amino acid sequence of the encoded 17906 protein, without altering the functional ability of the 17906 protein. For example, nucleotide substitutions leading to amino acid substitutions at "non-essential" amino acid residues can be made in the sequence of SEQ ID NO:1. A "non-essential" amino acid residue is a residue that can be altered from the wild-type sequence of 17906 *(e.g.,* the sequence of SEQ ID NO:2) without altering the biological activity, whereas an "essential" amino acid residue is required for biological activity. For example, amino acid residues that are conserved among the 17906 proteins of the present disclosuren are predicted to be particularly unamenable to alteration. Furthermore, additional amino acid residues that are conserved between the 17906 proteins of the present disclosure and other members of the G protein-coupled receptor family are not likely to be amenable to alteration.

Accordingly, the methods of the invention may include the use of nucleic acid molecules encoding 17906 proteins that contain changes in amino acid residues that are not essential for activity. Such 17906 proteins differ in amino acid sequence from SEQ ID NO:2, yet retain biological activity. In one embodiment, the isolated nucleic acid molecule comprises a nucleotide sequence encoding a protein, wherein the protein comprises an amino acid sequence at least about 30%, 35%, 40%, 45%, 50%, 55%, 60%, 65%, 70%, 75%, 80%, 85%, 90%, 95%, 98% or more identical to SEQ ID NO:2.

An isolated nucleic acid molecule encoding a 17906 protein identical to the protein of SEQ ID NO:2 can be created by introducing one or more nucleotide substitutions, additions or deletions into the nucleotide sequence of SEQ ID NO:1 such that one or more amino acid substitutions, additions or deletions are introduced into the encoded protein. Mutations can be introduced into SEQ ID NO:1 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e*.*g*., lysine, arginine, histidine), acidic side chains (*e*.*g*., aspartic acid, glutamic acid), uncharged polar side chains (*e*.*g*., glycine, asparagine, glutamine, serine, threonine, tyrosine, cysteine), nonpolar side chains (*e*.*g*., alanine, valine, leucine, isoleucine, proline, phenylalanine, methionine, tryptophan), beta-branched side chains (*e*.*g*., threonine, valine, isoleucine) and aromatic side chains (*e*.*g*., tyrosine, phenylalanine, tryptophan, histidine). Thus, a predicted nonessential amino acid residue in a 17906 protein is preferably replaced with another amino acid residue from the same side chain family. Alternatively, in another embodiment, mutations can be introduced randomly along all or part of a 17906 coding sequence, such as by saturation mutagenesis, and the resultant mutants can be screened for 17906 biological activity to identify mutants that retain activity. Following mutagenesis of SEQ ID NO:1, the encoded protein can be expressed recombinantly and the activity of the protein can be determined.

In a preferred embodiment, a mutant 17906 protein can be assayed for the ability to (1) interact with a non-17906 protein molecule, *e*.*g*., a 17906 ligand or substrate; (2) activate a 17906-dependent signal transduction pathway; or (3) modulate cell proliferation and/or migration mechanisms, or modulate the expression of cell surface adhesion molecules.

In addition to the nucleic acid molecules encoding 17906 proteins described herein, another aspect of the disclosure pertains to isolated nucleic acid molecules which are antisense thereto. An "antisense" nucleic acid comprises a nucleotide sequence which is complementary to a "sense" nucleic acid encoding a protein, *e*.*g*., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire 17906 coding strand, or to only a portion thereof. In one embodiment, an antisense nucleic acid molecule is antisense to a "coding region" of the coding strand of a nucleotide sequence encoding 17906. The term "coding region" refers to the region of the nucleotide sequence comprising codons which are translated into amino acid residues (*e*.*g*., the coding region of human 17906 corresponds to nucleotides 8-1273 of SEQ ID NO:1). In another embodiment, the antisense nucleic acid molecule is antisense to a "noncoding region" of the coding strand of a nucleotide sequence encoding 17906. The term "noncoding region" refers to 5' and 3' sequences which flank the coding region that are not translated into amino acids (*i*.*e*., also referred to as 5' and 3' untranslated regions).

Given the coding strand sequences encoding 17906 disclosed herein (*e*.*g*., nucleotides 8-1273 of SEQ ID NO:1), antisense nucleic acids of the disclosure can be designed according to the rules of Watson and Crick base pairing. The antisense nucleic acid molecule can be complementary to the entire coding region of 17906 mRNA, but more preferably is an oligonucleotide which is antisense to only a portion of the coding or noncoding region of 17906 mRNA. For example, the antisense oligonucleotide can be complementary to the region surrounding the translation start site of 17906 mRNA. An antisense oligonucleotide can be, for example, about 5, 10, 15, 20, 25, 30, 35, 40, 45 or 50 nucleotides in length. An antisense nucleic acid of the disclosure can be constructed using chemical synthesis and enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest).

In yet another embodiment, the 17906 nucleic acid molecules of the present disclosure can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4 (1): 5-23). As used herein, the terms "peptide nucleic acids" or "PNAs" refer to nucleic acid mimics, *e*.*g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al*. (1996) *supra*; Perry-O'Keefe et al. Proc. Natl. Acad. Sci. 93: 14670-675.

PNAs of 17906 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 17906 nucleic acid molecules can also be used in the analysis of single base pair mutations in a gene, (*e*.*g*., by PNA-directed PCR clamping); as 'artificial restriction enzymes' when used in combination with other enzymes, (*e*.*g*., S1 nucleases (Hyrup B. (1996) *supra*)); or as probes or primers for DNA sequencing or hybridization (Hyrup B. *et al.* (1996) *supra*; Perry-O'Keefe *supra*).

In another embodiment, PNAs of 17906 can be modified, (*e*.*g*., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of 17906 nucleic acid molecules can be generated which may combine the advantageous properties of PNA and DNA. Such chimeras allow DNA recognition enzymes, (e.g., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B. (1996) *supra*). The synthesis of PNA-DNA chimeras can be performed as described in Hyrup B. (1996) *supra* and Finn P.J. et al. (1996) Nucleic Acids Res. 24 (17): 3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag, M. et al. (1989) Nucleic Acid Res. 17: 5973-88). PNA monomers are then coupled in a stepwise manner to produce a chimeric molecule with a 5' PNA segment and a 3' DNA segment (Finn P.J. *et al.* (1996) *supra*). Alternatively, chimeric molecules can be synthesized with a 5' DNA segment and a 3' PNA segment (Peterser, K.H. et al. (1975) Bioorganic Med. Chem. Lett. 5: 1119-11124).

In other embodiments, the oligonucleotide may include other appended groups such as peptides (e.g., for targeting host cell receptors *in vivo*), or agents facilitating transport across the cell membrane (see, *e*.*g*., Letsinger et al. (1989) Proc. Natl. Acad. Sci. USA 86:6553-6556; Lemaitre et al. (1987) Proc. Natl. Acad. Sci. USA 84:648-652; PCT Publication No. W088/09810) or the blood-brain barrier (see, *e.g.,* PCT Publication No. W089/10134). In addition, oligonucleotides can be modified with hybridization-triggered cleavage agents (See, *e.g.,* Krol et al. (1988) Bio-Techniques 6:958-976) or intercalating agents. (See, *e*.*g*., Zon (1988) Pharm. Res. 5:539-549). To this end, the oligonucleotide may be conjugated to another molecule, (*e*.*g*., a peptide, hybridization triggered cross-linking agent, transport agent, or hybridization-triggered cleavage agent).

### Isolated 17906 Proteins and Anti-17906 Antibodies

The methods of the invention include the use of isolated 17906 proteins, and biologically active portions thereof, as well as polypeptide fragments suitable for use as immunogens to raise anti-17906 antibodies.

Isolated proteins of the present disclosure, preferably 17906 proteins, have an amino acid sequence sufficiently identical to the amino acid sequence of SEQ ID NO:2, or are encoded by a nucleotide sequence sufficiently identical to SEQ ID NO:1. As used herein, the term "sufficiently identical" refers to a first amino acid or nucleotide sequence which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least 30%, 40%, or 50% homology, preferably 60% homology, more preferably 70%-80%, and even more preferably 90-95% homology across the amino acid sequences of the domains and contain at least one and preferably two structural domains or motifs, are defined herein as sufficiently identical. Furthermore, amino acid or nucleotide sequences which share at least 30%, 40%, or 50%, preferably 60%, more preferably 70-80%, or 90-95% homology and share a common functional activity are defined herein as sufficiently identical.

As used interchangeably herein, a "17906 activity", "biological activity of 17906" or "functional activity of 17906", refers to an activity exerted by a 17906 protein, polypeptide or nucleic acid molecule on a 17906 responsive cell (*e*.*g*., a breast or ovarian cell) or tissue, or on a 17906 protein substrate, as determined *in vivo,* or *in vitro,* according to standard techniques. In one embodiment, a 17906 activity is a direct activity, such as an association with a 17906 target molecule. As used herein, a "target molecule" or "binding partner" is a molecule with which a 17906 protein binds or interacts in nature, such that 17906-mediated function is achieved. A 17906 target molecule can be a non-17906 molecule or a 17906 protein or polypeptide of the present disclosure. In an exemplary embodiment, a 17906 target molecule is a 17906 ligand. Alternatively, a 17906 activity is an indirect activity, such as a cellular signaling activity mediated by interaction of the 17906 protein with a 17906 ligand. Preferably, a 17906 activity is the ability to act as a signal transduction molecule and to modulate breast, or ovarian cell proliferation, differentiation, and/or migration. Accordingly, another embodiment of the disclosure features isolated 17906 proteins and polypeptides having a 17906 activity.

In one embodiment, native 17906 proteins can be isolated from cells or tissue sources by an appropriate purification scheme using standard protein purification techniques. In another embodiment, 17906 proteins are produced by recombinant DNA techniques. Alternative to recombinant expression, a 17906 protein or polypeptide can be synthesized chemically using standard peptide synthesis techniques.

An "isolated" or "purified" protein or biologically active portion thereof is substantially free of cellular material or other contaminating proteins from the cell or tissue source from which the 17906 protein is derived, or substantially free from chemical precursors or other chemicals when chemically synthesized. The language "substantially free of cellular material" includes preparations of 17906 protein in which the protein is separated from cellular components of the cells from which it is isolated or recombinantly produced. In one embodiment, the language "substantially free of cellular material" includes preparations of 17906 protein having less than about 30% (by dry weight) of non-17906 protein (also referred to herein as a "contaminating protein"), more preferably less than about 20% of non-17906 protein, still more preferably less than about 10% of non-17906 protein, and most preferably less than about 5% non-17906 protein. When the 17906 protein or biologically active portion thereof is recombinantly produced, it is also preferably substantially free of culture medium, *i*.*e*., culture medium represents less than about 20%, more preferably less than about 10%, and most preferably less than about 5% of the volume of the protein preparation.

The language "substantially free of chemical precursors or other chemicals" includes preparations of 17906 protein in which the protein is separated from chemical precursors or other chemicals which are involved in the synthesis of the protein. In one embodiment, the language "substantially free of chemical precursors or other chemicals" includes preparations of 17906 protein having less than about 30% (by dry weight) of chemical precursors or non-17906 chemicals, more preferably less than about 20% chemical precursors or non-17906 chemicals, still more preferably less than about 10% chemical precursors or non-17906 chemicals, and most preferably less than about 5% chemical precursors or non-17906 chemicals.

As used herein, a "biologically active portion" of a 17906 protein includes a fragment of a 17906 protein which participates in an interaction between a 17906 molecule and a non-17906 molecule. Biologically active portions of a 17906 protein include peptides comprising amino acid sequences sufficiently identical to or derived from the amino acid sequence of the 17906 protein, e.g., the amino acid sequence shown in SEQ ID NO:2, which include less amino acids than the full length 17906 protein, and exhibit at least one activity of a 17906 protein. Typically, biologically active portions comprise a domain or motif with at least one activity of the 17906 protein, *e*.*g*., modulating cell proliferation mechanisms. A biologically active portion of a 17906 protein can be a polypeptide which is, for example, 10, 25, 50, 100, 200, or more amino acids in length. Biologically active portions of a 17906 protein can be used as targets for developing agents which modulate a 17906 mediated activity, *e*.*g*., a cell proliferation mechanism. A biologically active portion of a 17906 protein comprises a protein in which regions of the protein are deleted, can be prepared by recombinant techniques and evaluated for one or more of the functional activities of a native 17906 protein.

In a preferred embodiment, the 17906 protein has an amino acid sequence shown in SEQ ID NO:2. In other embodiments, the 17906 protein is substantially identical to SEQ ID NO:2, and retains the functional activity of the protein of SEQ ID NO:2, yet differs in amino acid sequence due to natural allelic variation or mutagenesis, as described in detail in subsection I above. Accordingly, in another embodiment, the 17906 protein is a protein which comprises an amino acid sequence at least about 30%, 35%, 40%, 45%, 50%, 55%, 60%, 65%, 70%, 75%, 80%, 85%, 90%, 95%, 98% or more identical to SEQ ID NO:2.

To determine the percent identity of two amino acid sequences or of two nucleic acid sequences, the sequences are aligned for optimal comparison purposes (*e.g.,* gaps can be introduced in one or both of a first and a second amino acid or nucleic acid sequence for optimal alignment and non-identical sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence (*e*.*g*., when aligning a second sequence to the 17906 amino acid sequence of SEQ ID NO:2 having 516 amino acid residues, at least 136, preferably at least 181, more preferably at least 227, even more preferably at least 272, and even more preferably at least 317, 362 or 408 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (J. Mol. Biol. (48):444-453 (1970)) algorithm which has been incorporated into the GAP program in the GCG software package (available at http://www.gcg.com), using either a Blosum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (Myers and Miller, Comput. Appl. Biosci. 4:11-17 (1988)) which has been incorporated into the ALIGN program (version 2.0), using a PAM120 weight residue table, a gap length penalty of 12 and a gap penalty of 4.

The nucleic acid and protein sequences of the present disclosure can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to 17906 nucleic acid molecules of the disclosure. BLAST protein searches can be performed with the XBLAST program, score = 100, wordlength = 3 to obtain amino acid sequences homologous to 17906 protein molecules of the disclosure. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e*.*g*., XBLAST and NBLAST) can be used. See http://www.ncbi.nlm.nih.gov.

The methods of the invention may also use 17906 chimeric or fusion proteins. As used herein, a 17906 "chimeric protein" or "fusion protein" comprises a 17906 polypeptide operatively linked to a non-17906 polypeptide. A "17906 polypeptide" refers to a polypeptide having an amino acid sequence corresponding to 17906, whereas a "non-17906 polypeptide" refers to a polypeptide having an amino acid sequence corresponding to a protein which is not substantially homologous to the 17906 protein, *e*.*g*., a protein which is different from the 17906 protein and which is derived from the same or a different organism. Within a 17906 fusion protein the 17906 polypeptide can correspond to all or a portion of a 17906 protein. In a preferred embodiment, a 17906 fusion protein comprises at least one biologically active portion of a 17906 protein. In another preferred embodiment, a 17906 fusion protein comprises at least two biologically active portions of a 17906 protein. Within the fusion protein, the term "operatively linked" is intended to indicate that the 17906 polypeptide and the non-17906 polypeptide are fused in-frame to each other. The non-17906 polypeptide can be fused to the N-terminus or C-terminus of the 17906 polypeptide.

Moreover, the 17906-fusion proteins of the disclosure can be used as immunogens to produce anti-17906 antibodies in a subject, to purify 17906 ligands and in screening assays to identify molecules which inhibit the interaction of 17906 with a 17906 substrate.

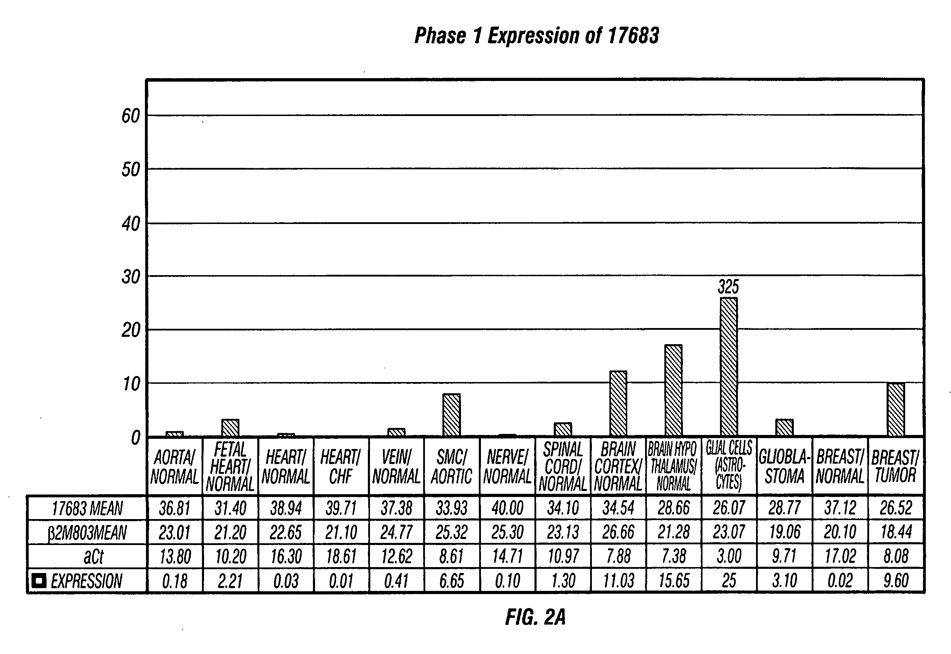

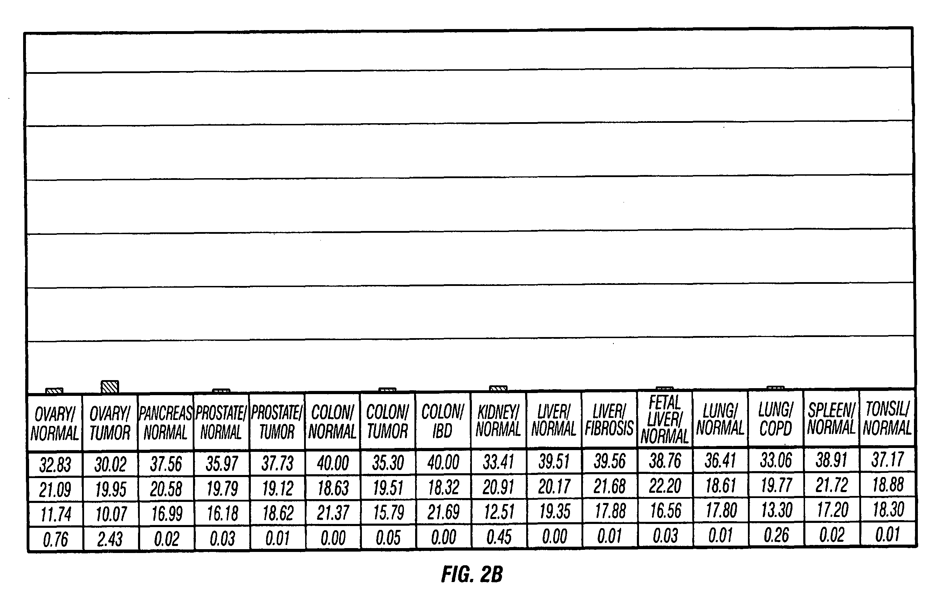

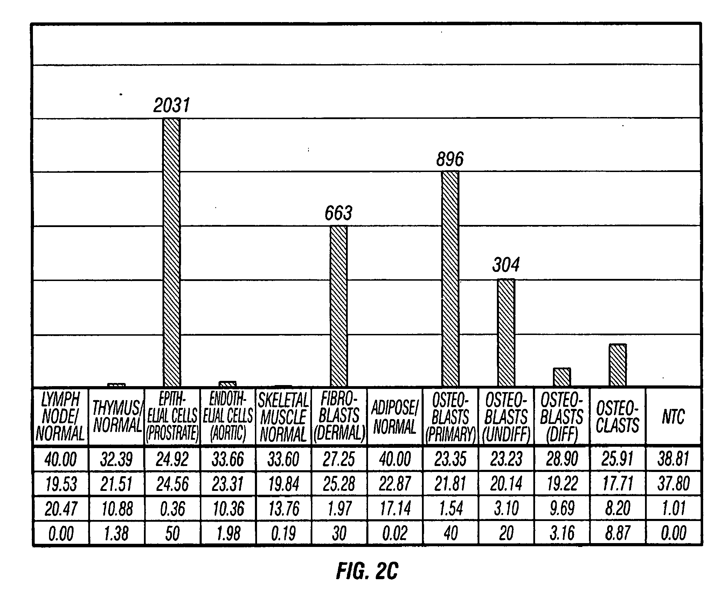

An isolated 17906 protein, or a portion or fragment thereof, can be used as an immunogen to generate antibodies that bind 17906 using standard techniques for polyclonal and monoclonal antibody preparation. A full-length 17906 protein can be used or, alternatively, the disclosure provides antigenic peptide fragments of 17906 for use as immunogens. The antigenic peptide of 17906 comprises at least 8 amino acid residues of the amino acid sequence shown in SEQ ID NO:2 and encompasses an epitope of 17906 such that an antibody raised against the peptide forms a specific immune complex with 17906. Preferably, the antigenic peptide comprises at least 10 amino acid residues, more preferably at least 15 amino acid residues, even more preferably at least 20 amino acid residues, and most preferably at least 30 amino acid residues. Preferred epitopes encompassed by the antigenic peptide are regions of 17906 that are located on the surface of the protein, e.g., hydrophilic regions, as well as regions with high antigenicity (see Figure 2).

A 17906 immunogen typically is used to prepare antibodies by immunizing a suitable subject, (e.g., rabbit, goat, mouse or other mammal) with the immunogen. An appropriate immunogenic preparation can contain, for example, recombinantly expressed 17906 protein or a chemically synthesized 17906 polypeptide. The preparation can further include an adjuvant, such as Freund's complete or incomplete adjuvant, or similar immunostimulatory agent. Immunization of a suitable subject with an immunogenic 17906 preparation induces a polyclonal anti-17906 antibody response.

Accordingly, another aspect of the disclosure pertains to anti-17906 antibodies. The term "antibody" as used herein refers to immunoglobulin molecules and immunologically active portions of immunoglobulin molecules, *i*.*e*., molecules that contain an antigen binding site which specifically binds (immunoreacts with) an antigen, such as 17906. Examples of immunologically active portions of immunoglobulin molecules include F(ab) and F(ab')2 fragments which can be generated by treating the antibody with an enzyme such as pepsin. The disclosure provides polyclonal and monoclonal antibodies that bind 17906. The term "monoclonal antibody" or "monoclonal antibody composition", as used herein, refers to a population of antibody molecules that contain only one species of an antigen binding site capable of immunoreacting with a particular epitope of 17906. A monoclonal antibody composition thus typically displays a single binding affinity for a particular 17906 protein with which it immunoreacts.

Polyclonal anti-17906 antibodies can be prepared as described above by immunizing a suitable subject with a 17906 immunogen. The anti-17906 antibody titer in the immunized subject can be monitored over time by standard techniques, such as with an enzyme linked immunosorbent assay (ELISA) using immobilized 17906. If desired, the antibody molecules directed against 17906 can be isolated from the mammal (e.g., from the blood) and further purified by well known techniques, such as protein A chromatography to obtain the IgG fraction. At an appropriate time after immunization, *e*.*g*., when the anti-17906 antibody titers are highest, antibody-producing cells can be obtained from the subject and used to prepare monoclonal antibodies by standard techniques, such as the hybridoma technique originally described by Kohler and Milstein (1975) Nature 256:495-497) (see also, Brown et al. (1981) J. Immunol. 127:539-46; Brown et al. (1980) J. Biol. Chem .255:4980-83; Yeh et al. (1976) Proc. Natl. Acad. Sci. USA 76:2927-31; and Yeh et al. (1982) Int. J. Cancer 29:269-75), the more recent human B cell hybridoma technique (Kozbor *et al*. (1983) *Immunol Today* 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a 17906 immunogen as described above, and the culture supernatants of the resulting hybridoma cells are screened to identify a hybridoma producing a monoclonal antibody that binds 17906.

Any of the many well known protocols used for fusing lymphocytes and immortalized cell lines can be applied for the purpose of generating an anti-17906 monoclonal antibody (see, *e.g.,* G. Galfre et al. (1977) Nature 266:55052; Gefter et al. Somatic Cell Genet., cited supra; Lerner, Yale J. Biol. Med., cited supra; Kenneth, *Monoclonal Antibodies*, cited *supra*). Moreover, the ordinarily skilled worker will appreciate that there are many variations of such methods which also would be useful. Typically, the immortal cell line (*e.g.,* a myeloma cell line) is derived from the same mammalian species as the lymphocytes. For example, murine hybridomas can be made by fusing lymphocytes from a mouse immunized with an immunogenic preparation of the present disclosure with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the disclosuren are detected by screening the hybridoma culture supernatants for antibodies that bind 17906, *e*.*g*., using a standard ELISA assay.

Alternative to preparing monoclonal antibody-secreting hybridomas, a monoclonal anti-17906 antibody can be identified and isolated by screening a recombinant combinatorial immunoglobulin library (*e*.*g*., an antibody phage display library) with 17906 to thereby isolate immunoglobulin library members that bind 17906. Kits for generating and screening phage display libraries are commercially available (*e*.*g*., the Pharmacia *Recombinant Phage Antibody System*, Catalog No. 27-9400-01; and the Stratagene *SurfZ4P*^{™}*Phage Display Kit,* Catalog No. 240612). Additionally, examples of methods and reagents particularly amenable for use in generating and screening antibody display library can be found in, for example, *Ladner et al*. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J 12:725-734; Hawkins et al. (1992) J. Mol. Biol. 226:889-896; Clarkson et al. (1991) Nature 352:624-628; Gram et al. (1992) Proc. Natl. Acad. Sci. USA 89:3576-3580; Garrad et al. (1991) Bio/Technology 9:1373-1377; Hoogenboom et al. (1991) Nuc. Acid Res. 19:4133-4137; Barbas et al. (1991) Proc. Natl. Acad. Sci. USA 88:7978-7982; and McCafferty et al. Nature (1990) 348:552-554.

Additionally, recombinant anti-17906 antibodies, such as chimeric and humanized monoclonal antibodies, comprising both human and non-human portions, which can be made using standard recombinant DNA techniques, can also be used in the methods of the present invention. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, M., European Patent Application 171,496; Morrison *et al.* European Patent Application 173,494; Neuberger et al. PCT International Publication No. WO 86/01533; Cabilly et al. U.S. Patent No. 4,816,567; Cabilly *et al.* European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Canc. Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) BioTechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

An anti-17906 antibody (*e*.*g*., monoclonal antibody) can be used to isolate 17906 by standard techniques, such as affinity chromatography or immunoprecipitation. An anti-17906 antibody can facilitate the purification of natural 17906 from cells and of recombinantly produced 17906 expressed in host cells. Moreover, an anti-17906 antibody can be used to detect 17906 protein (*e*.*g*., in a cellular lysate or cell supernatant) in order to evaluate the abundance and pattern of expression of the 17906 protein. Anti-17906 antibodies can be used diagnostically to monitor protein levels in tissue as part of a clinical testing procedure, *e*.*g*., to, for example, determine the efficacy of a given treatment regimen. Detection can be facilitated by coupling (*i.e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include
¹²⁵I, ¹³¹I, ³⁵S or ³H.

This invention is further illustrated by the following examples which should not be construed as limiting.

### EXAMPLES

### Example 1: Identification and Characterization of Human 17906 cDNAs

The human 17906 sequence (Figure 1A; SEQ ID NO:1), which is approximately 2795 nucleotides long including untranslated regions, contains a predicted methionine-initiated coding sequence (SEQ ID NO:3; Figure 1B) of about 1266 nucleotides (nucleotides 8 to 1273 of SEQ ID NO:1). The coding sequence encodes a 422 amino acid protein (SEQ ID NO:2; Figure 1B).

### EXAMPLE 2: Expression and Tissue Distribution of 17906 mRNA

Northern blot hybridizations with various RNA samples can be performed under standard conditions and washed under stringent conditions, i.e., 0.2xSSC at 65°C. A DNA probe corresponding to all or a portion of the 17906 cDNA (SEQ ID NOs:1 or 3) can be used. The DNA is radioactively labeled with 32P-dCTP using the Prime-It Kit (Stratagene, La Jolla, CA) according to the instructions of the supplier. Filters containing mRNA from mouse hematopoietic and endocrine tissues, and cancer cell lines (Clontech, Palo Alto, CA) can be probed in ExpressHyb hybridization solution (Clontech) and washed at high stringency according to manufacturer's recommendations.

TaqMan real-time quantitative RT-PCR was used to detect the presence of RNA transcript corresponding to human 17906 in several tissues. It was found that the corresponding orthologs of 17906 are expressed in a variety of tissues. The results of this screening are shown in Figures 2-9.

Reverse Transcriptase PCR (RT-PCR) was used to detect the presence of RNA transcript corresponding to human 17906 in RNA prepared from cells and tissues related to osteoblasts. Expression of 17906 was assessed in several tissues. A relatively low expression of the transcript was found in differentiated osteoblasts, and relatively high expression of the transcript was found in primary cultured osteoblasts.

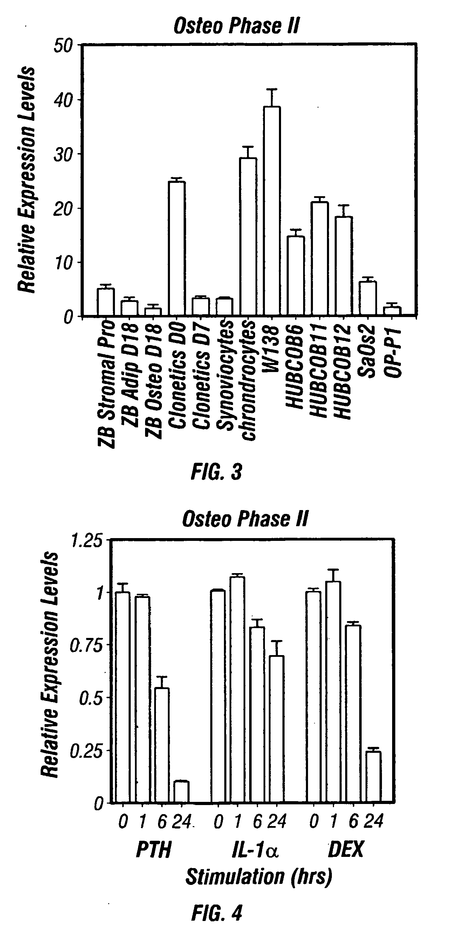

Relative expression levels of the 17906 was assessed in osteogenic cells and adipogenic cells using TaqMan PCR. The results of this comparison are shown in Figure 3.

Figures 2 and 3 also depict results of TaqMan PCR used to assess the expression of 17906 in several cellular models of osteoporosis.

Relative mRNA expression levels of the 17906 gene was also assessed in osteoblasts stimulated with parathyroid hormone (PTH), interleukin-1α (IL-1α), and dexamethasone (DEX) as shown in Figure 4.

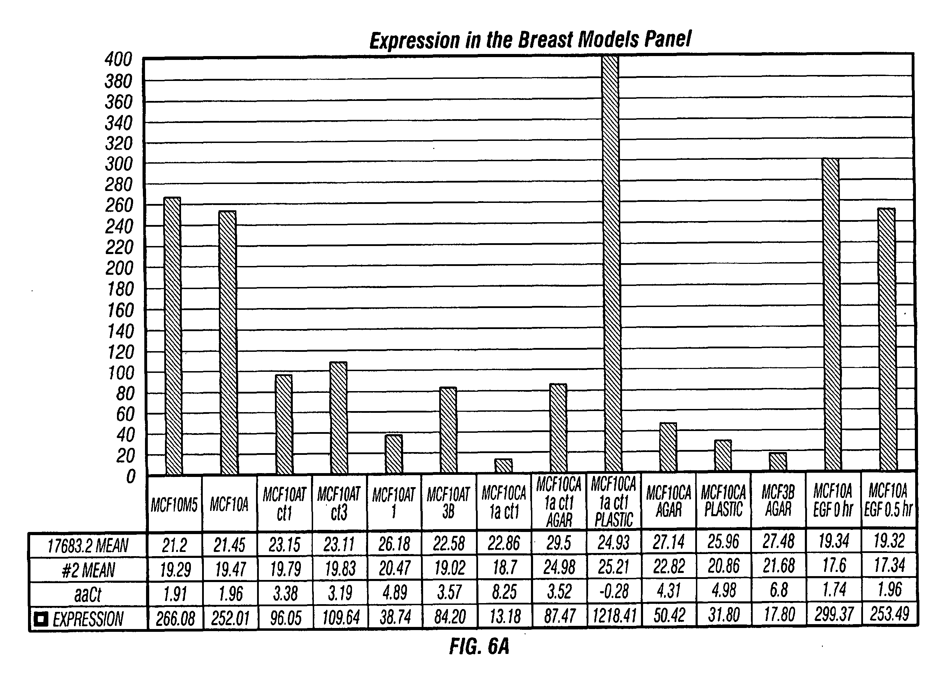

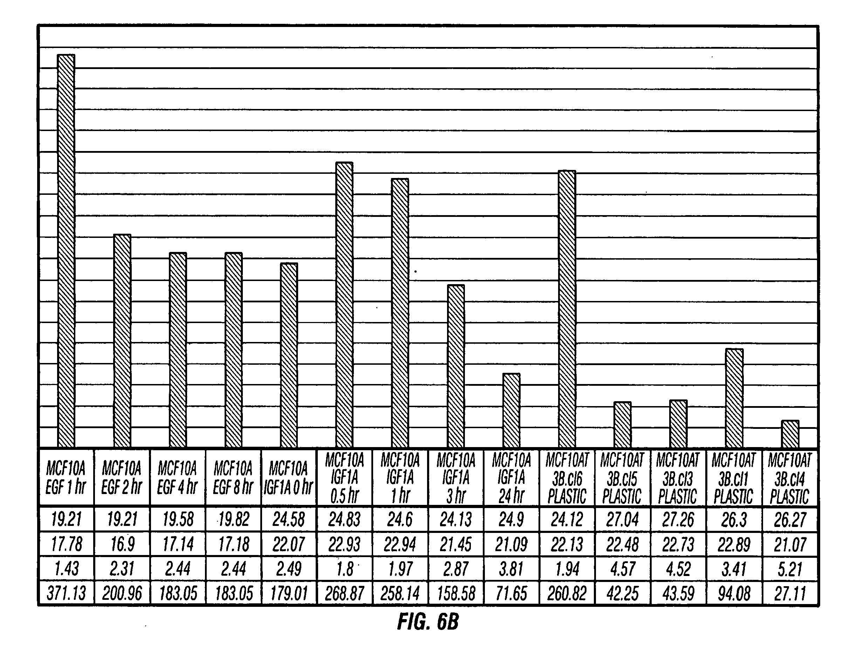

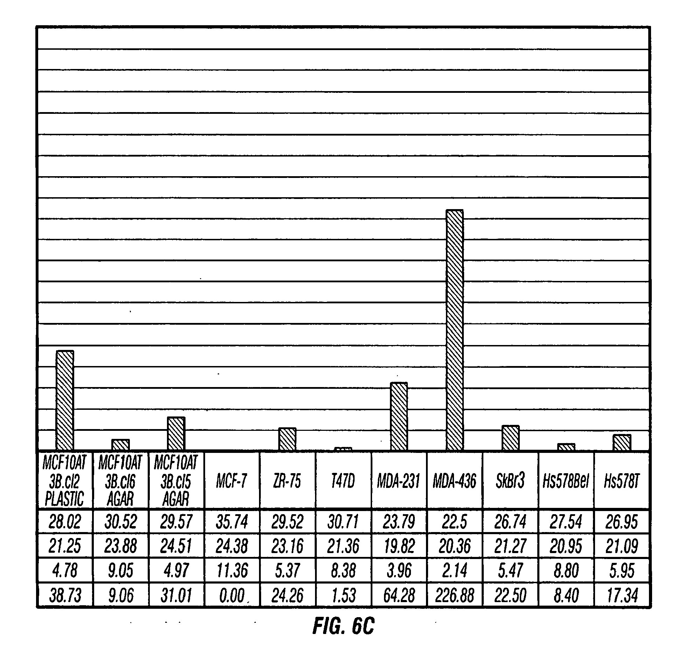

Reverse Transcriptase PCR (RT-PCR) was used to detect the presence of RNA transcript corresponding to human 17906 in RNA prepared from tumor and normal tissues. Figures 6-9 illustrate the relative expression levels and tissue distribution of the 17906 genes in various tissues using Taq Man PCR. If a subject has a disease characterized by underexpression or overexpression of a 17906 gene, modulators which have a stimulatory or inhibitory effect on carboxypeptidase activity (e.g., carboxypeptidase gene expression) can be administered to individuals to treat (prophylactically or therapeutically) carboxypeptidase-associated disorders.

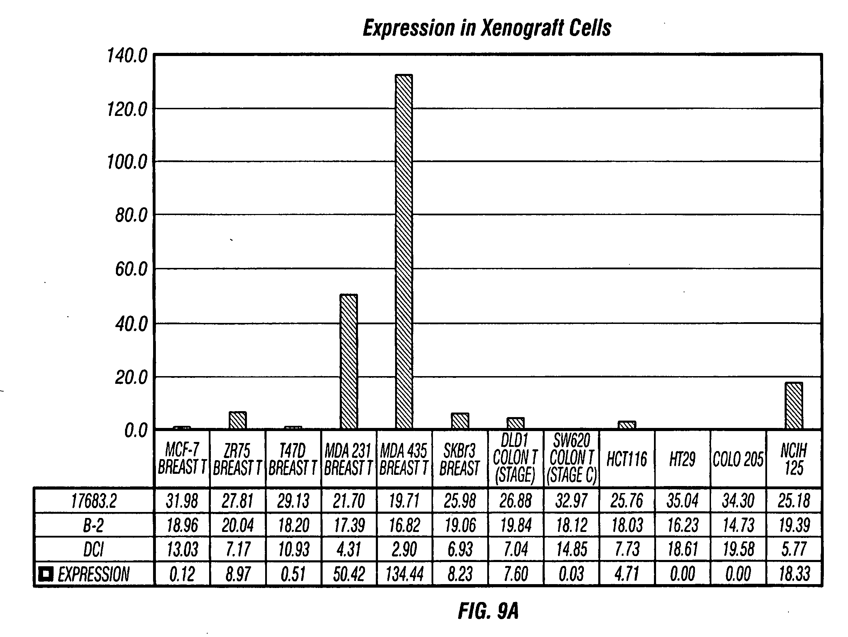

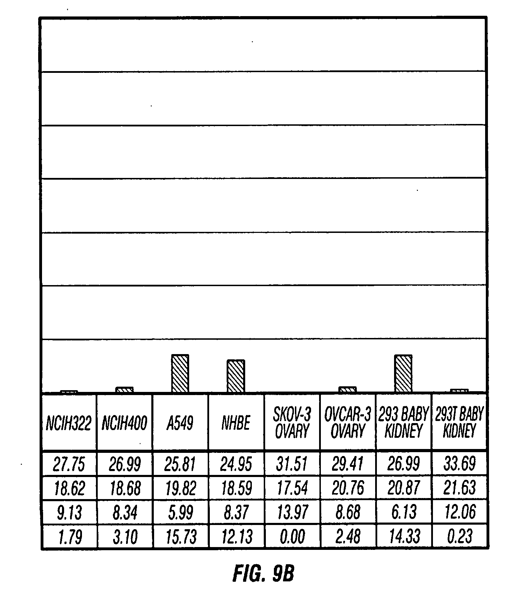

Figure 8 illustrates the relative expression levels of 17906 in various tissues using TaqMan PCR, and significant expression in prostate epithelial cells. Variable expression was found in xenographs of cell lines tested as shown in Figure 9 for 17906; the highest expression for 17906 was found in MDA-435 breast tumor cell line.

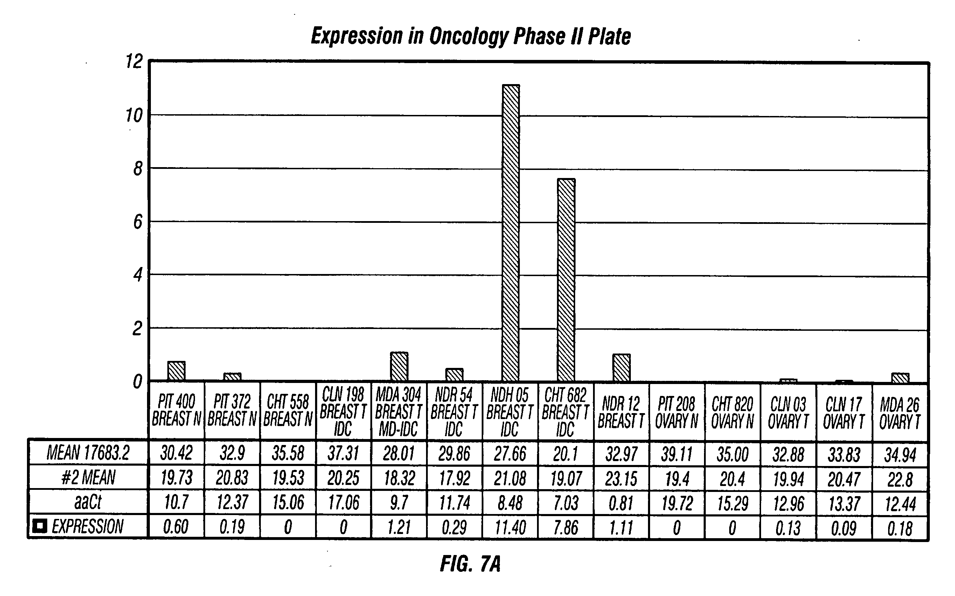

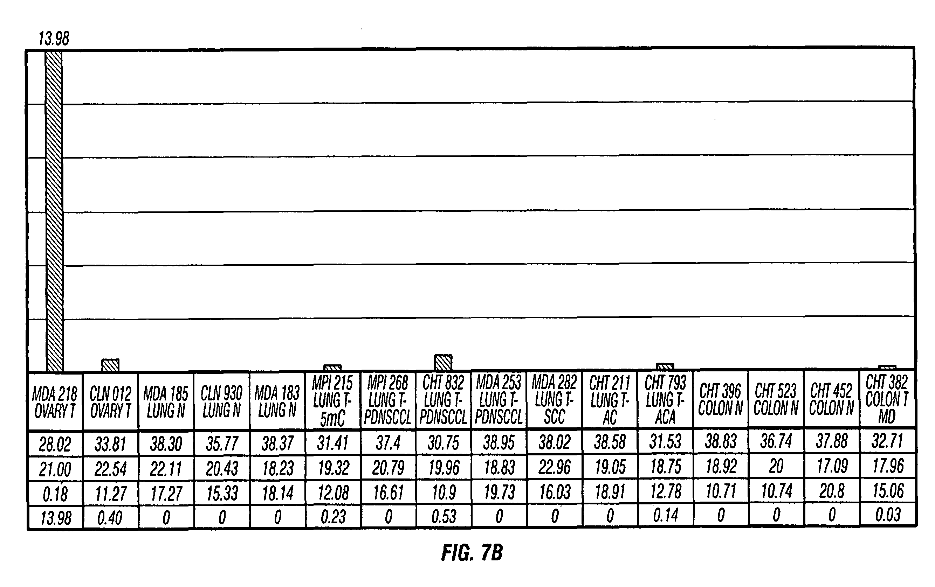

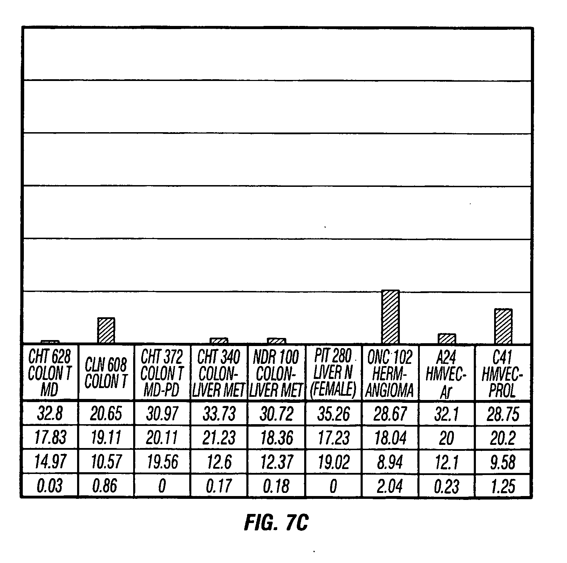

Figure 6 shows significant expression in the MCF10A (m25 Plastic) breast model. Figure 7 shows some 17906 expression of 17906 mRNA relative to a no template control showing an increased expression in 4/6 breast tumors in comparison to normal breast tissues, 5/5 ovarian tumors in comparison to normal ovarian tissues, 3/7 lung tumors in comparison to normal lung tissues, and 3/4 colon tumors and 2/2 colon metastases in comparison to normal colon tissues. Again, expression was detected using Taq Man analysis.

As seen by these results, 17906 molecules have been found to be overexpressed in some tumors or cells, where the molecules may be inappropriately propagating either cell proliferation or cell survival signals or have aberrant protein kinase activity. As such, 17906 molecules may serve as specific and novel identifiers of such tumor cells or disorders.

Further, modulators of the 17906 molecules are useful for the treatment of cancer. For example, inhibitors of the 17906 molecules are useful for the treatment of cancer where 17906 is upregulated in tumor cells such as ovarian, colon, breast, and lung cancer and colon metastases, and are useful as a diagnostic.

### EXAMPLE 3: EXPRESSION OF RECOMBINANT 17906 PROTEIN IN BACTERIAL CELLS

In this example, 17906 is expressed as a recombinant glutathione-S-transferase (GST) fusion polypeptide in E. coli and the fusion polypeptide is isolated and characterized. Specifically, 17906 is fused to GST and this fusion polypeptide is expressed in E. coli, e.g., strain PEB199. Expression of the GST-17906, fusion protein in PEB 199 is induced with IPTG. The recombinant fusion polypeptide is purified from crude bacterial lysates of the induced PEB199 strain by affinity chromatography on glutathione beads. Using polyacrylamide gel electrophoretic analysis of the polypeptide purified from the bacterial lysates, the molecular weight of the resultant fusion polypeptide is determined.

### Example 4: Expression of Recombinant 17906 Protein in COS Cells

To express the 17906 gene in COS cells, the pcDNA/Amp vector by Invitrogen Corporation (San Diego, CA) is used. This vector contains an SV40 origin of replication, an ampicillin resistance gene, an E. coli replication origin, a CMV promoter followed by a polylinker region, and an SV40 intron and polyadenylation site. A DNA fragment encoding the entire 17906 protein and an HA tag (Wilson et al. (1984) Cell 37:767) or a FLAG tag fused in-frame to its 3' end of the fragment is cloned into the polylinker region of the vector, thereby placing the expression of the recombinant protein under the control of the CMV promoter.

To construct the plasmid, the 17906 DNA sequence is amplified by PCR using two primers. The 5' primer contains the restriction site of interest followed by approximately twenty nucleotides of the 17906 coding sequence starting from the initiation codon; the 3' end sequence contains complementary sequences to the other restriction site of interest, a translation stop codon, the HA tag or FLAG tag and the last 20 nucleotides of the 17906 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 17906 gene is inserted in the correct orientation. The ligation mixture is transformed into E. coli cells (strains HB101, DH5a, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the 17906-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989. The expression of the 17906 polypeptide is detected by radiolabelling (35S-methionine or 35S-cysteine available from NEN, Boston, MA, can be used) and immunoprecipitation (Harlow, E. and Lane, D. Antibodies: A Laboratory Manual, Cold Spring Harbor Laboratory Press, Cold Spring Harbor, NY, 1988) using an HA specific monoclonal antibody. Briefly, the cells are labeled for 8 hours with 35S-methionine (or 35S-cysteine). The culture media are then collected and the cells are lysed using detergents (RIPA buffer, 150 mM NaCl, 1% NP-40,0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 17906 coding sequence is cloned directly into the polylinker of the pCDNA/Amp vector using the appropriate restriction sites. The resulting plasmid is transfected into COS cells in the manner described above, and the expression of the 17906 polypeptide is detected by radiolabelling and immunoprecipitation using a 17906 specific monoclonal antibody.

## Claims

1. A method of identifying a subject having breast or ovarian cancer or at risk for developing breast or ovarian cancer comprising:
a) contacting a first sample obtained from the subject comprising nucleic acid molecules with a hybridization probe comprising at least 25 contiguous nucleotides of SEQ ID NO:1;
b) detecting the presence of a nucleic acid molecule in the first sample that hybridizes to the probe,
c) contacting a second sample obtained from a control subject comprising nucleic acid molecules with a hybridization probe comprising at least 25 contiguous nucleotides of SEQ ID NO:1;
d) detecting the presence of a nucleic acid molecule in the second sample that hybridizes to the probe; and
e) comparing the level of nucleic acid molecules which hybridize to the probe in the first sample with the level of nucleic acids which hybridize to the probe in the second sample,
wherein an increased level of nucleic acids which hybridize in the first sample relative to the second sample identifies a subject having breast or ovarian cancer or at risk for developing breast or ovarian, cancer.

2. The method of claim 1, wherein said hybridization probe is detectably labeled.

3. The method of claim 2, wherein said sample comprising nucleic acid molecules is subjected to agarose gel electrophoresis and southern blotting prior to contacting with said hybridization probe.

4. The method of claim 1, wherein said sample comprising nucleic acid molecules is subjected to agarose gel electrophoresis and northern blotting prior to contacting with said hybridization probe.

5. The method of claim 1, wherein said detecting is by *in situ* hybridization.

6. A method of identifying a subject having breast or ovarian, or at risk for developing breast or ovarian cancer comprising:
a) contacting a first sample obtained from the subject comprising nucleic acid molecules with a first and a second amplification primer, the first primer comprising at least 25 contiguous nucleotides of SEQ ID NO:1 and the second primer comprising at least 25 contiguous nucleotides from the complement of SEQ ID NO:1;
b) incubating the first sample under conditions that allow nucleic acid amplification;
c) contacting a second sample obtained from a control subject comprising nucleic acid molecules with a first and a second amplification primer, the first primer comprising at least 25 contiguous nucleotides of SEQ ID NO:1 and the second primer comprising at least 25 contiguous nucleotides from the complement of SEQ ID NO:1;
d) incubating the second sample under conditions that allow nucleic acid amplification; and
e) comparing the level of nucleic acid amplification in the first sample relative to the level of nucleic acid amplification in the second sample,
wherein an increased level of nucleic acid amplification in the first sample relative to the second sample identifies a subject having breast or ovarian cancer or at risk for developing breast or ovarian cancer.

7. The method of claim 6, wherein said sample comprising nucleic acid molecules is subjected to agarose gel electrophoresis after said incubation step.

8. The method of any one of claims 1 or 6, wherein said method is used to detect mRNA in said sample.

9. The method of any one of claims 1 or 6, wherein said method is used to detect genomic DNA in said sample.

10. A method of identifying a subject having breast or ovarian cancer or at risk for developing breast or ovarian cancer comprising:
a) contacting a first sample obtained from the subject comprising polypeptides with an antibody which specifically binds to a polypeptide of SEQ ID NO:2;
b) detecting the presence of a polypeptide in the first sample that binds to the antibody;
c) contacting a second sample from a control subject comprising polypeptides with an antibody which specifically binds to a polypeptide of SEQ ID NO:2;
d) detecting the presence of a polypeptide in the second sample that binds to the antibody; and
e) comparing the level of antibody binding in the first sample relative to the level of antibody binding in the second sample,
wherein an increased level of antibody binding in the first sample relative to the second sample identifies a subject having breast or ovarian cancer or at risk for developing breast or ovarian cancer.

11. The method of claim 10, wherein said antibody is detectably labeled.

12. A method for identifying a compound capable of treating breast or ovarian cancer the method comprising assaying the ability of the compound to decrease expression of a nucleic acid molecule of SEQ ID NO:1 or to down regulate the activity of a polypeptide of SEQ ID NO:2, thereby identifying a compound capable of treating breast or ovarian cancer.

13. A method for evaluating the efficacy of a treatment of breast or ovarian cancer, in a sample isolated from a subject, being treated with a protocol under evaluation, which comprises:
assessing the expression level of a nucleic acid molecule selected from the group consisting of:
a) a nucleic acid molecule comprising a nucleotide sequence which is at least 80% identical to the nucleotide sequence of SEQ ID NO:1;
b) a nucleic acid molecule which encodes a polypeptide comprising the amino acid sequence of SEQ ID NO:2; and
c) a nucleic acid molecule comprising the nucleotide sequence of SEQ ID NO:1;
or assessing the activity of a polypeptide encoded by the nucleic acid molecule, wherein a decrease in the expression level of the nucleic acid or a decrease in the activity of the polypeptide after the treatment, relative to the level before the treatment, is indicative of the efficacy of the treatment of breast or ovarian cancer.

## Patentansprüche

1. Verfahren zum Identifizieren eines Subjekts mit Brust-oder Eierstockkrebs oder mit einem Risiko, Brust- oder Eierstockkrebs zu entwickeln, bei dem man
a) eine erste von dem Subjekt erhaltende Probe, die Nukleinsäuremoleküle umfasst, mit einer Hybridisierungssonde in Kontakt bringt, welche mindestens 25 zusammenhängende Nukleotide von SEQ ID NO:1 umfasst;
b) die Anwesenheit eines Nukleinsäuremoleküls, das an die Sonde hybridisiert, in der ersten Probe detektiert;
c) eine zweite von dem Subjekt erhaltende Probe, die Nukleinsäuremoleküle umfasst, mit einer Hybridisierungssönde in Kontakt bringt, welche mindestens 25 zusammenhängende Nukleotide von SEQ ID NO:1 umfasst;
d) die Anwesenheit eines Nukleinsäuremoleküls, das an die Sonde hybridisiert, in der zweiten Probe detektiert;
e) die Menge der Nukleinsäuremoleküle, die in der ersten Probe an die Sonde hybridisieren, mit der Menge der Nukleinsäuren vergleicht, die in der zweiten Probe an die Sonde hybridisieren,
wobei eine erhöhte Menge an Nukleinsäuren, die in der ersten Probe relativ zu der zweiten Probe hybridisieren, ein Subjekt mit Brust- oder Eierstockkrebs oder mit einem Risiko, Brust- oder Eierstockkrebs zu entwickeln, identifiziert.

2. Verfahren nach Anspruch 1, bei dem die Hybridisierungssonde detektierbar markiert ist.

3. Verfahren nach Anspruch 2, bei dem man die Probe, die Nukleinsäuremoleküle umfasst, einer Agarose-Gelelektrophorese und einem Southern-Blot unterzieht, bevor man sie mit der Hybridisierungssonde in Kontakt bringt.

4. Verfahren nach Anspruch 1, bei dem man die Probe, die Nukleinsäuremoleküle umfasst, einer Agarose-Gelelektrophorese und einem Northern-Blot unterzieht, bevor man sie mit der Hybridisierungssonde in Kontakt bringt.

5. Verfahren nach Anspruch 1, bei dem das Detektieren durch *in situ*-Hybridisierung erfolgt.

6. Verfahren zum Identifizieren eines Subjekts mit Brust-oder Eierstockkrebs oder mit einem Risiko, Brust- oder Eierstockkrebs zu entwickeln, bei dem man
a) eine erste von dem Subjekt erhaltende Probe, die Nukleinsäuremoleküle umfasst, mit einem ersten und einem zweiten Amplifikationsprimer in Kontakt bringt, wobei der erste Primer mindestens 25 zusammenhängende Nukleotide von SEQ ID NO:1 umfasst und der zweite Primer mindestens 25 zusammenhängende Nukleotide vom Komplement von SEQ ID NO:1 umfasst;
b) die erste Probe unter Bedingungen inkubiert, die eine Nukleinsäure-Amplifikation ermöglichen;
c) eine zweite von einem Kontrollsubjekt erhaltende Probe, die Nukleinsäuremoleküle umfasst, mit einem ersten und einem zweiten Amplifikationsprimer in Kontakt bringt, wobei der erste Primer mindestens 25 zusammenhängende Nukleotide von SEQ ID NO:1 umfasst und der zweite Primer mindestens 25 zusammenhängende Nukleotide vom Komplement von SEQ ID NO:1 umfasst;
d) die zweite Probe unter Bedingungen inkubiert, die eine Nukleinsäure-Amplifikation ermöglichen, und
e) das Ausmaß der Nukleinsäure-Amplifikation in der ersten Probe mit dem Ausmaß der Nukleinsäure-Amplifikation in der zweiten Probe vergleicht,
wobei eine erhöhtes Ausmaß an Nukleinsäure-Amplifikation in der ersten Probe relativ zu der zweiten Probe ein Subjekt mit Brust- oder Eierstockkrebs oder mit einem Risiko, Brust- oder Eierstockkrebs zu entwickeln, identifiziert.

7. Verfahren nach Anspruch 6, bei dem man die Probe, die Nukleinsäuremoleküle umfasst, nach dem Inkubationsschritt einer Agarose-Gelelektrophorese unterzieht.

8. Verfahren nach einem der Ansprüche 1 bis 6, bei dem man das Verfahren zum Detektieren von mRNA in der Probe verwendet.

9. Verfahren nach einem der Ansprüche 1 bis 6, bei dem das Verfahren zum Detektieren von genomischer DNA in der Probe verwendet.

10. Verfahren zum Identifizieren eines Subjekts mit Brust-oder Eierstockkrebs oder mit einem Risiko, Brust- oder Eierstockkrebs zu entwickeln, bei dem man
a) eine erste von dem Subjekt erhaltende Probe, die Polypeptide umfasst, mit einem Antikörper in Kontakt bringt, der spezifisch an ein Polypeptid gemäß SEQ ID NO:2 bindet;
b) die Anwesenheit eines Polypeptids, das an den Antikörper bindet, in der ersten Probe detektiert;
c) eine zweite von einem Kontrollsubjekt erhaltende Probe, die Polypeptide umfasst, mit einem Antikörper in Kontakt bringt, der spezifisch an ein Polypeptid gemäß SEQ ID NO:2 bindet;
d) die Anwesenheit eines Polypeptids, das an den Antikörper bindet, in der zweiten Probe detektiert;
e) das Ausmaß der Antikörperbindung in der ersten Probe mit dem Ausmaß der Antikörperbindung in der zweiten Probe vergleicht,
wobei ein erhöhtes Ausmaß an Antikörperbindung in der ersten Probe relativ zu der zweiten Probe ein Subjekt mit Brust- oder Eierstockkrebs oder mit einem Risiko, Brust-oder Eierstockkrebs zu entwickeln, identifiziert.

11. Verfahren nach Anspruch 10, bei dem der Antikörper detektierbar markiert ist.

12. Verfahren zum Identifizieren einer Verbindung, die Brust-oder Eierstockkrebs behandeln kann, bei dem man in einem Assay die Fähigkeit der Verbindung bewertet, die Expression eines Nukleinsäuremoleküls gemäß SEQ ID NO:1 zu verringern oder die Aktivität eines Polypeptids gemäß SEQ ID NO:2 herunterzuregeln, wodurch eine Verbindung identifiziert wird, die Brust- oder Eierstockkrebs behandeln kann.

13. Verfahren zur Bewertung der Wirksamkeit einer Behandlung von Brust- oder Eierstockkrebs in einer Probe, die aus einem Subjekt isoliert wurde, das mit einem zu bewertenden Protokoll behandelt wird, bei dem man
das Ausmaß der Expression eines Nukleinsäuremoleküls bewertet, das ausgewählt ist aus der Gruppe bestehend aus:
a) einem Nukleinsäuremolekül, das eine Nukleotidsequenz umfasst, welche mindestens zu 80 % mit der Nukleotidsequenz von SEQ ID NO:1 identisch ist,
b) einem Nukleinsäuremolekül, das für ein Polypeptid kodiert, welches die Aminosäuresequenz von SEQ ID NO:2 umfasst, und
c) einem Nukleinsäuremolekül, das die Nukleotidsequenz von SEQ ID NO:1 umfasst,
oder die Aktivität eines Polypeptids bewertet, das von dem Nukleinsäuremolekül kodiert wird, wobei eine Abnahme im Ausmaß der Expression der Nukleinsäure oder eine Abnahme der Aktivität des Polypeptids nach der Behandlung relativ zum Ausmaß vor der Behandlung die Wirksamkeit der Behandlung von Brust- oder Eierstockkrebs anzeigt.

## Revendications

1. Procédé d'identification d'un sujet ayant un cancer du sein ou ovarien ou présentant un risque de développer un cancer du sein ou ovarien, comprenant :
a) mettre en contact un premier échantillon obtenu à partir du sujet comprenant des molécules d'acide nucléique avec une sonde d'hybridation comprenant au moins 25 nucléotides contigus de SEQ ID NO:1 ;
b) détecter la présence d'une molécule d'acide nucléique dans le premier échantillon qui s'hybride à la sonde ;
c) mettre en contact un second échantillon obtenu à partir d'un sujet témoin comprenant des molécules d'acide nucléique avec une sonde d'hybridation comprenant au moins 25 nucléotides contigus de SEQ ID NO:1 ;
d) détecter la présence d'une molécule d'acide nucléique dans le second échantillon qui s'hybride à la sonde ; et
e) comparer le taux de molécules d'acide nucléique qui s'hybrident à la sonde dans le premier échantillon avec le taux d'acides nucléiques qui s'hybrident à la sonde dans le second échantillon,
dans lequel un taux accru d'acides nucléiques qui s'hybrident dans le premier échantillon par rapport au second échantillon identifie un sujet ayant un cancer du sein ou ovarien ou présentant un risque de développer un cancer du sein ou ovarien.

2. Procédé selon la revendication 1, dans lequel ladite sonde d'hybridation est marquée de façon détectable.

3. Procédé selon la revendication 2, dans lequel ledit échantillon comprenant des molécules d'acide nucléique est soumis à une électrophorèse sur gel d'agarose et à un transfert de type Southern avant la mise en contact avec ladite sonde d'hybridation.

4. Procédé selon la revendication 1, dans lequel ledit échantillon comprenant des molécules d'acide nucléique est soumis à une électrophorèse sur gel d'agarose et à un transfert de type Northern avant la mise en contact avec ladite sonde d'hybridation.

5. Procédé selon la revendication 1, dans lequel ladite détection s'effectue par hybridation *in situ*.

6. Procédé d'identification d'un sujet ayant un cancer du sein ou ovarien ou présentant un risque de développer un cancer du sein ou ovarien, comprenant :
a) mettre en contact d'un premier échantillon obtenu à partir du sujet comprenant des molécules d'acide nucléique avec une première et une seconde amorce d'amplification, la première amorce comprenant au moins 25 nucléotides contigus de SEQ ID NO:1 et la seconde amorce comprenant au moins 25 nucléotides contigus provenant du complément de SEQ ID NO:1 ;
b) faire incuber le premier échantillon dans des conditions qui permettent une amplification de l'acide nucléique ;
c) mettre en contact un second échantillon obtenu à partir d'un sujet témoin comprenant des molécules d'acide nucléique avec une première et une seconde amorce d'amplification, la première amorce comprenant au moins 25 nucléotides contigus de SEQ ID NO:1 et la seconde amorce comprenant au moins 25 nucléotides contigu provenant du complément de SEQ ID NO:1 ;
d) faire incuber le second échantillon dans des conditions qui permettent une amplification d'acide nucléique ; et
e) comparer le taux d'amplification d'acide nucléique dans le premier échantillon au taux d'amplification d'acide nucléique dans le second échantillon,
dans lequel un taux accru d'amplification d'acide nucléique dans le premier échantillon par rapport au second échantillon identifie un sujet ayant un cancer du sein ou ovarien ou présentant un risque de développer un cancer du sein ou ovarien.

7. Procédé selon la revendication 6, dans lequel ledit échantillon comprenant des molécules d'acide nucléique est soumis à une électrophorèse sur gel d'agarose après ladite étape d'incubation.

8. Procédé selon l'une quelconque des revendications 1 ou 6, dans lequel ledit procédé est utilisé pour détecter l'ARNm dans ledit échantillon.

9. Procédé selon l'une quelconque des revendications 1 ou 6, dans lequel ledit procédé est utilisé pour détecter l'ADN génomique dans ledit échantillon.

10. Procédé d'identification d'un sujet ayant un cancer du sein ou ovarien ou présentant un risque de développer un cancer du sein ou ovarien, comprenant :
a) mettre en contact un premier échantillon obtenu à partir du sujet comprenant des polypeptides avec un anticorps qui se lie de façon spécifique à un polypeptide de SEQ ID NO:2 ;
b) détecter la présence d'un polypeptide dans le premier échantillon qui se lie à l'anticorps ;
c) mettre en contact un second échantillon provenant d'un sujet témoin comprenant des polypeptides avec un anticorps qui se lie de façon spécifique à un polypeptide de SEQ ID NO:2 ;
d) détecter la présence d'un polypeptide dans le second échantillon qui se lie à l'anticorps ; et
e) comparer le niveau de liaison à l'anticorps dans le premier échantillon au niveau de liaison à l'anticorps dans le second échantillon,
dans lequel un taux accru de liaison à l'anticorps dans le premier échantillon par rapport au second échantillon identifie un sujet ayant un cancer du sein ou ovarien ou présentant un risque de développer un cancer du sein ou ovarien.

11. Procédé selon la revendication 10, dans lequel ledit anticorps est marqué de façon détectable.

12. Procédé d'identification d'un composé capable de traiter un cancer du sein ou ovarien, le procédé comprenant l'essai de l'aptitude du composé à diminuer l'expression dudit molécule d'acide nucléique de SEQ ID NO:1 ou à réguler négativement l'activité d'un polypeptide de SEQ ID NO:2, permettant ainsi d'identifier un composé capable de traiter un cancer du sein ou ovarien.

13. Procédé d'évaluation de l'efficacité d'un traitement du cancer du sein ou ovarien, dans un échantillon isolé à partir d'un sujet, qui est traité par un protocole sous évaluation, qui comprend :
évaluer le taux d'expression d'une molécule d'acide nucléique choisie dans le groupe constitué par :
a) une molécule d'acide nucléique comprenant une séquence nucléotidique qui est au moins à 80 % identique à la séquence nucléotidique de SEQ ID NO:1 ;
b) une molécule d'acide nucléique qui code pour un polypeptide comprenant la séquence d'acides aminés SEQ ID NO:2 ; et
c) une molécule d'acide nucléique comprenant la séquence nucléotidique de SEQ ID NO:1 ;
ou évaluer l'activité d'un polypeptide codé par la molécule d'acide nucléique,
dans lequel une diminution du taux d'expression de l'acide nucléique ou une diminution de l'activité du polypeptide après le traitement, par rapport au taux avant le traitement, est indicative de l'efficacité du traitement du cancer du sein ou ovarien.
